# EUROPEAN PATENT SPECIFICATION

(11) **EP 4 081 803 B1**
(45) Date of publication and mention of the grant of the patent: **22.10.2025**
(21) Application number: 20838477.6
(22) Date of filing: 18.12.2020
(51) Int. Cl.: G01N 33/68

(54) **NEW PROTEIN MARKERS OF RENAL DAMAGE**
NEUE PROTEINMARKER FÜR NIERENSCHÄDEN
NOUVEAUX MARQUEURS DE PROTÉINE DE LÉSION RÉNALE

(30) Priority: 23.12.2019 EP 19383190
(43) Date of publication of application: 02.11.2022
(73) Proprietor: Fundació Institut d'Investigació en Ciències de la Salut Germans Trias i Pujol (IGTP), 08916 Badalona (ES)
(72) Inventor: BORRÀS SERRES, Francisco E., 08916 BADALONA (ES); LAUZURICA VALDEMOROS, L. Ricardo, 08916 BADALONA (ES); CARRERAS PLANELLA, Laura, 08916 BADALONA (ES)
(74) Representative: Hoffmann Eitle
(86) International application number: PCT/EP2020/087290
(87) International publication number: WO 2021/130138

(56) References cited:
- WO-A1-2013/078253
- WO-A1-2013/158777
- KR-A- 20190 094 967
- J T REILLY ET AL: "Vitronectin (serum spreading factor): its localisation in normal and fibrotic tissue.", JOURNAL OF CLINICAL PATHOLOGY, vol. 41, no. 12, 1 December 1988 (1988-12-01), GB, pages 1269 - 1272, XP055692250, ISSN: 0021-9746, DOI: 10.1136/jcp.41.12.1269
- BARBARA M. KLINKHAMMER ET AL: "Treatment of Renal Fibrosis-Turning Challenges into Opportunities", ADVANCES IN CHRONIC KIDNEY DISEASE, vol. 24, no. 2, 1 March 2017 (2017-03-01), USA, pages 117 - 129, XP055459587, ISSN: 1548-5595, DOI: 10.1053/j.ackd.2016.11.002

## Description

### FIELD OF THE INVENTION

The present invention relates to the field of diagnostic markers of renal damage. Specifically, relates to urine protein markers for determining the degree of fibrosis in a kidney and methods using thereof. In particular, it relates to methods using said biomarkers in patient's monitoring patient's suffering from chronic kidney disease or further to renal transplantation. It further pertains to associated second medical uses, methods of treatment, kits and use thereof in the methods of the invention.

### BACKGROUND OF THE INVENTION

Chronic kidney disease (CKD) is characterized by a decrease of renal function associated to a loss of glomerular filtration that progressed over months or years that can eventually lead to end stage renal disease (ESRD). Patients with ESRD will require a renal replacement therapy that can either be dialysis or kidney transplantation. Dialysis is considered to be a temporary option while patients wait for kidney transplantation, which is the treatment of choice because it increases patient survival and quality of life, apart from being less costly than dialysis (Merion et al. 2018).

The introduction of immunosuppressive drugs decades ago reduced greatly the frequency of acute rejection (Marcén 2009). However, following solid organ transplantation, immunosuppressive drugs must be taken indefinitely to prevent graft loss, adverse effects of the therapy contributing to different types of morbidity of patients after transplantation and treatment election. General dosing parameters derived from epidemiological data and clinical parameters of each patient are used as guidance for immunosuppressive regimen as an attempt to provide a personalized treatment. Nevertheless, a graft rejection rate of about 10% per year after transplantation has been reported (Gondos et al. 2013; Ucar, Demir, and Sever 2018; Lamb, Lodhi, and Meier-Kriesche 2011). In particular, chronic rejection is the main pathological state that accounts for most graft losses (Meier-Kriesche, Schold, and Kaplan 2004). In fact, in the United States kidney transplant failure is the fourth cause of ESRD. The causes for long-term renal graft loses are diverse - from dysregulated fibrosis to drug toxicity, or antibody mediated rejection (Sellarés et al. 2012).

Accordingly, further to renal transplantation, patients require periodic follow up even in the absence of signs of kidney failure (Williams et al. 2012). Monitoring of the renal graft is performed by determining the serum creatinine concentration, the estimated glomerular filtration rate, and proteinuria at different time intervals, depending on the time since the transplant, and following the international guidelines of the Kidney Disease Improving Global Outcomes (KDIGO) group (Kidney Disease: Improving Global Outcomes (KDIGO) Transplant Work Group 2009). These guidelines define chronic graft dysfunction as a clinical-pathological entity characterized by an estimated glomerular filtration rate below 40 ml/min/1,72m² and a proteinuria greater than 500 mg/day from the first year post-transplant. Patients are routinely monitored for their creatinine levels every 2-3 months and yearly for proteinuria after the first year post-transplant. Thus, increased serum creatinine, decreased glomerular filtration rate and the appearance of proteinuria suggest renal graft dysfunction.

Current tests for diagnosing graft rejection are far from being ideal. Classic markers of renal injury, like urine and serum creatinine levels, estimated glomerular filtration (eGFR) or total urine protein excretion, are easily measurable but these have been reported to be late markers, non-specific and to show a low predictiveness for rejection. Serum creatinine is the most commonly used clinical marker for the estimation of GFR. Creatinine levels derive from the metabolism of creatine in skeletal muscle. It is released constantly into the circulation and filtered freely through the glomeruli, without being reabsorbed or metabolized at the renal level. Thus, Creatinine levels depend on production, and muscle mass, but also by hydration and excretion rate, age, sex, and weight. The onset of the increase in serum creatinine is often associated with a histological lesion already established, irreversible, and without the possibility of therapeutic intervention (Chapman, O'Connell, and Nankivell 2005; K. Solez, Vincenti, and Filo 1998). It is well-established that the persistence or new appearance of proteinuria is a sign of graft damage (Shamseddin and Knoll 2011) and of possible graft-loss (Reichel, Zeier, and Ritz 2004). WO 2013/078253 A1 discloses new candidate proteins as markers of kidney injury in the diagnosis, risk stratification, prognosis, classifying and monitoring of the renal status of the subject.

Renal biopsy is the gold standard test in the diagnosis of the pathology affecting the transplanted kidney, and it is nowadays essential to establish a correct diagnosis and an appropriate therapeutic treatment (Al-Awwa, Hariharan, and First 1998). However, it also presents some drawbacks, it has low reproducibility and foremost it is a highly invasive technique which involves an increased risk of complications. Moreover, the process of kidney rejection is not only complex and variable amongst patients but also dynamic in time. Several injuries can occur concomitantly, each of them affecting one or more structures on the kidney. However, kidney biopsies only enable to visualize a small portion of the kidney. An adequate biopsy must contain at least 10 glomeruli and 2 arteries, a small fraction from the estimated one million glomeruli contained in a kidney. Thus, the fact that the sample is obtained from a small part of the kidney is why, even when an "adequate" kidney biopsy is passed over to the pathologist, there is no assurance that the portion of tissue collected is a representation of the events occurring in the whole organ.

Therefore, a renal biopsy presents an inherent limitation associated to organ representation which may lead to misdiagnosis.

Accordingly, there is a clear need for non-invasive biomarkers of kidney damage or pathological phenotype which would help in reducing the number of required biopsies, thus improving the patient's welfare and decreasing the risk of complications. Non-invasive markers will also allow a more frequent monitoring of the renal function after transplantation, overall improving graft survival.

There is also a need for non-invasive biomarkers for determining the best therapeutic option according to the underlying pathological phenotype and/or its degree of severity (Lo, Kaplan, and Kirk 2014).

Fibrosis is defined as the replacement of functional tissue parenchyma with extracellular matrix (ECM). Fibrosis of a transplanted kidney predominantly affects the tubulointerstitium which represents 90% of kidney volume. Accordingly, the terms "fibrosis" and "interstitial fibrosis/tubular atrophy (IFTA)" have been used interchangeably herein. IFTA can be the result of various renal insults, such as ischemia-reperfusion injury, rejection, infection and pharmacological treatment (e.g., anticalcineurinic agents). Thus, as stated by Vanhove et al., IFTA cannot be considered a disease but the final common end point of several disease processes (Vanhove, Goldschmeding, and Kuypers 2017).

There are limited treatment options for patients with IFTA. The inability to diagnose IFTA early, before irreversible ECM accumulation, limits the development of therapeutics. Accordingly, it is critically important to be able to identify those patients with a low-moderate degree of fibrosis which would likely benefit from the treatment with antifibrotic agents.

Moreover, the degree of IFTA has been reported to have prognostic value. In particular, IFTA of the kidney allograft was described to associate with worse renal function (Schwarz et al. 2005; Fernando G. Cosio, Grande, Larson, et al. 2005) and predict functional decline as well as graft survival (Fernando G. Cosio, Grande, Larson, et al. 2005; Nankivell et al. 2001; Grimm et al. 2003; Roos-van Groningen et al. 2006; Servais et al. 2011). Remarkably, IFTA has been associated with worse prognosis independently of the underlying pathological phenotype (eg, T cell-mediated rejection and antibody-mediated rejection) (Naesens et al. 2014; John et al. 2010). Yet only moderate to severe fibrosis has been reported to be predictive of outcome whereas mild fibrosis, even in extended followed-up patients, has not been found to associate with prognosis (Vanhove, Goldschmeding, and Kuypers 2017; Serón et al. 2002; Pape et al. 2003; Fernando G. Cosio, Grande, Wadei, et al. 2005; F. G. Cosio et al. 2016).

Thus, there is a need of non-invasive biomarkers which levels are associated with the degree of fibrosis in the renal transplant, in particular to identify those patients presenting a moderate to severe degree of kidney fibrosis.

Mohammed-Ali Zahraa et al. (Mohammed Ali Zahraa et al. 2019) investigated 6 proteins previously identified to be regulated by Angiotensin II (i.e. (thrombospondin-I [TSP1], ras homolog family member B [RHOB], bone marrow stromal cell antigen 1 [BST1], lysophospholipase I [LYPLA1], glutamine synthetase [GLNA], and laminin subunit beta-2 [LAMB2]) as non-invasive markers of fibrosis in kidney transplant recipients. The authors reported that urine excretion of the six Angll signature proteins was increased in patients with IFTA with respect to controls. These urine proteins were found to be more informative than total urine protein excretion rate. In particular, the combined use of the proteins was able to predict IFTA and control groups with an AUC of 0.86. Individual proteins showing best discrimination values were BST1 (AUC: 0.71), RHOB (AUC = 0.80), LYPLA1 (AUC = 0.81), and TSP1_TIV (AUC = 0.80). Thus, this document teaches the ability of this signature to diagnose fibroses (absence vs mild fibrosis). However, this document is silent on the ability of these proteins to differentiate between diverse degrees of fibrosis in the kidney parenchyma, for instance between absence-mild fibrosis and moderate-high fibrosis which has been described to have a high prognostic value.

KR 2019 0094967 A mentions transglutaminase 2 (TG2) and interleukine-6 (IL-6) levels as potential biomarkers for classifying a kidney transplanted patient according to their IFTA degree. A correlation between IFTA severity and TG2 or IL-6 was only found further to normalization by creatinine concentration which as mentioned above is a value which levels in urine may depend on many variables.

Kidney vitronectin (Vtn) mRNA and protein levels were described by López-Guisa et al. 2011 to be increased in a mice model of chronic kidney disease with respect to the sham group. The authors further investigated the pathological role of VTN in fibrogenesis and found that the overall degree of renal fibrosis was similar between Vtn +/+ and Vtn -/- mice at all investigated time points leading them to conclude that VTN minimally impacts fibrogenesis. J T Reilly et al 1988 describes the accumulation of VTN in various tissues affected of fibrosis. This document does not show any evidence of distinct levels of VTN due to different degrees of fibrosis in those tissues. This document is also not concerned with kidney fibrosis.

WO 2013158777A1 relates to a method for identifying a kidney transplant recipient at an increased risk of developing interstitial fibrosis or tubular atrophy which comprises determining the levels of clusterin in a urine sample and comparing the level of clusterin in the patient sample to the level of clusterin in a control sample from the urine of a non-fibrotic kidney transplant recipient. Accordingly, this document is concerned with the likelihood of development of fibrosis and does not provide however a method to assess the present degree or severity of fibrosis in the kidney of the subject.

Extracellular vesicles (EV) are 50 to 200 nm lipid-bilayered vesicles which are produced by practically all cells in the organism and can be found in most biological fluids. Interestingly, their content varies upon the cell of origin and its physiological state (Yáñez-Mó et al. 2015) , which is the reason why during the last decade, EV have emerged as promising reservoirs of biomarkers (Lo, Kaplan, and Kirk 2014). In the nephrology field, numerous groups have been studying proteins, mRNA or microRNA found in urinary EV (uEV) as promising biomarkers of kidney alterations since they may reflect the state of cells composing the excretory system such as renal epithelial cells, including glomerular podocytes, renal tubule cells and the cells lining the urinary drainage system (Gámez-Valero et al. 2015; Salih et al. 2016; Pocsfalvi et al. 2015; Alvarez et al. 2013; Zhang et al. 2017). For instance, the concentration of neutrophil gelatinase-associated lipocalin (NGAL) in urinary extracellular vesicles (EVs) was proposed by Alvarez et al. as predictor of kidney dysfunction after renal transplantation (Alvarez et al. 2013).

### SUMMARY OF THE INVENTION

The present invention is as defined in the claims.

The inventors studied the proteomic content of uEV in urine samples from a first small cohort of kidney transplanted patients (n=23) using shotgun mass spectrometry. Urine samples were from patients previously classified into 4 groups based on the results of their biopsies. The histopathological findings were graded with a score from 0 to 3 following Banff criteria (Kim Solez et al. 1993; revisited: Haas et al. 2018). These 4 groups are: normal kidney function (NKF) (no alteration of analytical parameters) and three different pathological phenotypes, namely interstitial fibrosis/tubular atrophy (IFTA), acute cellular rejection (ACR) and calcineurin inhibitors toxicity (CNIT).

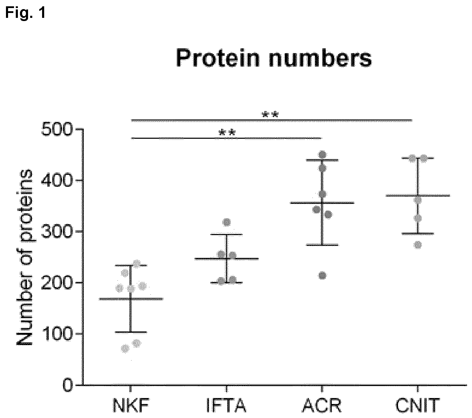

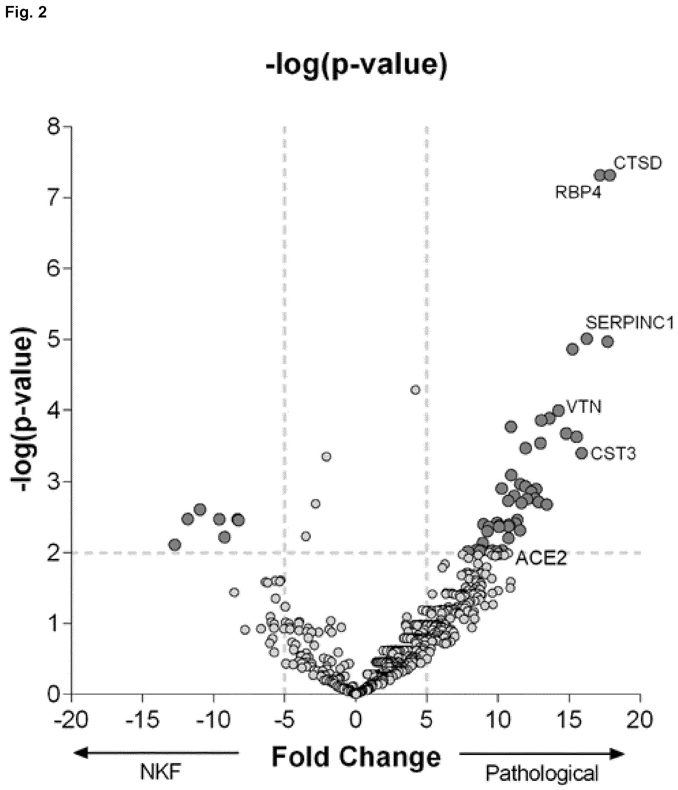

The results showed statistically significant differences in the number of proteins among patients suffering from ACR or CNIT compared to NKFs patients (Fig.1). Seven proteins were significantly more expressed in the NKF group, while 48 proteins were significantly more expressed in the pathological group (Fig.2). Some of these proteins were further analysed in a second cohort of patients (validation) from a different hospital and using a more specific technique (targeted proteomics).

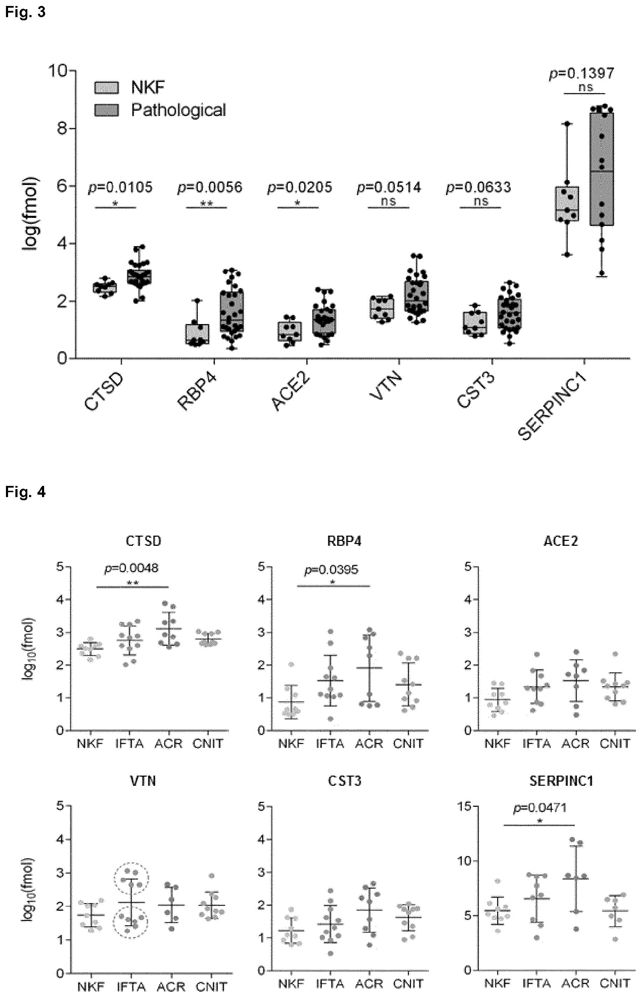

Targeted proteomics studies were conducted in the validation phase. This methodology is used for the identification of specific proteins within a complex sample background and is based on selected or multiple reaction monitoring (Uzozie et al. 2017; Picotti et al. 2010; Lange et al. 2008; Malmström et al. 2012). Three proteins, namely Cathepsin-D (CTSD), Retinol binding protein 4 (RBP4) and Angiotensin-converting enzyme 2 (ACE2) were selected in the validation phase for being significantly over-expressed in pathological patients compared to NKF (Figure 3).

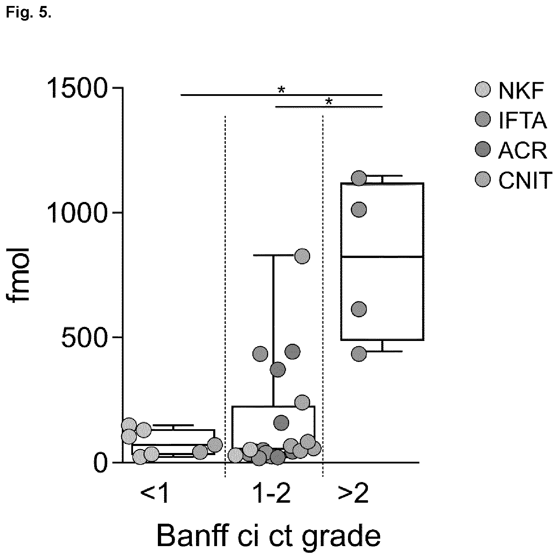

Besides, when analysing each of the markers according to the underlying kidney pathology, CTSD, RBP4 and SERPINC1 were shown to associate with ACR (Fig. 4). Vitronectin (VTN) did not enable to discriminate any of the patient groups with statistical significance (Fig. 4). However, the inventors realized that within the IFTA group two subgroups of individuals could be observed, namely those with high and low VTN levels. This observation led the inventors to investigate the existing relationship between VTN and fibrosis. Two of the parameters analysed with Banff criteria are chronic interstitial lesions and chronic tubular lesions (CICT), which are used to determine the grade of kidney fibrosis and IFTA. The inventors surprisingly found that VTN was associated to the degree of kidney fibrosis being useful for discriminating in a statistically significant manner low (<1 cict BANNF criteria) and moderate (1-2 cict BANNF criteria) from severe (>2 cict BANNF criteria) fibrosis as shown in Fig. 5. As mentioned above, being able to identify those patients with low-moderate fibrosis has a high value for clinicians since those patients can still benefit from a treatment against fibrosis.

One of the most important advantages of this panel of proteins over kidney biopsy, which is the current gold standard test for altered renal function diagnosis, is that these proteins can be measured in urine non-invasively and as often as desired. This panel of proteins could help clinicians in the monitoring of patients and in their decision making for choosing the most appropriate treatment, with the overall aim to extend graft and patient survival.

A first aspect of the invention relates to the *in vitro* use of VTN protein levels as kidney fibrosis biomarker. In a related aspect, the invention pertains to a method for the screening, diagnosis or monitoring of kidney fibrosis in a subject, wherein said method comprises:
a. determining vitronectin (VTN) protein levels in a urine sample from said subject; and
b. determining whether the VTN protein levels in the sample are increased with respect to a reference value;
wherein VTN protein levels in a) increased with respect to the reference value is indicative of fibrosis, preferably of moderate, severe or moderate-severe fibrosis.

In an additional aspect, the invention relates to a method for determining the prognosis of a subject on the basis of its degree of kidney fibrosis, preferably a kidney transplanted patient, wherein said method comprises
a. determining vitronectin (VTN) protein levels in a urine sample from said subject; and
b. determining whether the VTN protein levels in the sample are increased with respect to a reference value;
wherein VTN protein levels in a) increased with respect to the reference value is indicative of moderate, severe or moderate-severe fibrosis and poor prognosis, for instance it predicts future functional decline and in transplanted patients a higher risk of graft-loss.

It is described herein a method for selecting a subject which would likely benefit from a treatment suitable for treating renal damage or pathological phenotype, wherein said subject is selected for presenting renal damage or pathological phenotype according to a method as described herein above.

It is further described a method for treating a patient with renal damage or pathological phenotype by administering a therapeutically effective amount of a suitable treatment, wherein said patient has been selected as having renal damage or pathological phenotype by a method as described herein above.

In an additional aspect, the present invention concerns a method for selecting a subject which would likely benefit from the administration of an agent suitable for the preventive and/or therapeutic treatment of fibrosis (also referred herein as antifibrotic agent), wherein said patient is selected for presenting low or moderate fibrosis, as defined in the claims.

It is further described a kit for determining the levels of one or more of the target markers as described herein in a biological sample (preferably a urine sample) isolated from a subject.

In still a further aspect, the invention relates to the use of a kit as described above in any of the claimed methods, such as in a method for the screening, diagnosis or monitoring of kidney fibrosis in a subject; a method for selecting a subject which would likely benefit from the administration of an agent suitable for the preventive and/or therapeutic treatment of fibrosis and a method for determining the prognosis of a subject.

### BRIEF DESCRIPTION OF THE DRAWINGS

**Fig. 1****.** Number of different proteins per group. Kruskall-Wallis test (** p<0.001 ).
**Fig. 2****.** Volcano plot showing proteins significantly more expressed in pathological (IFTA, ACR and CNIT; to the right) and NKF (to the left) samples. Each dot represents a protein. Y-axis represents -log(p-value), where significant p-value=0.01 is indicated by a horizontal dashed grey line (two-sided t-test). Significant proteins with a fold change >5 or <-5 (indicated by vertical dashed grey lines on the x-axis) are shown with bigger darker circles. Those proteins discussed in this work in further detail are labelled with their gene name.
**Fig. 3****.** Expression in NKF compared to pathological samples of proteins in the validation cohort. Mann-Whitney test (significance p<0.05).
**Fig. 4****.** Expression in each group of proteins in the validation cohort. Kruskall-Wallis with Dunn's test for multiple comparison (significance *p<0.05, **p<0.005).
**Fig. 5****.** Vitronectin expression in different grades of kidney fibrosis. The expression of Kidney biopsies from the study patients were graded chronic interstitial lesions and chronic tubular lesions (CICT)

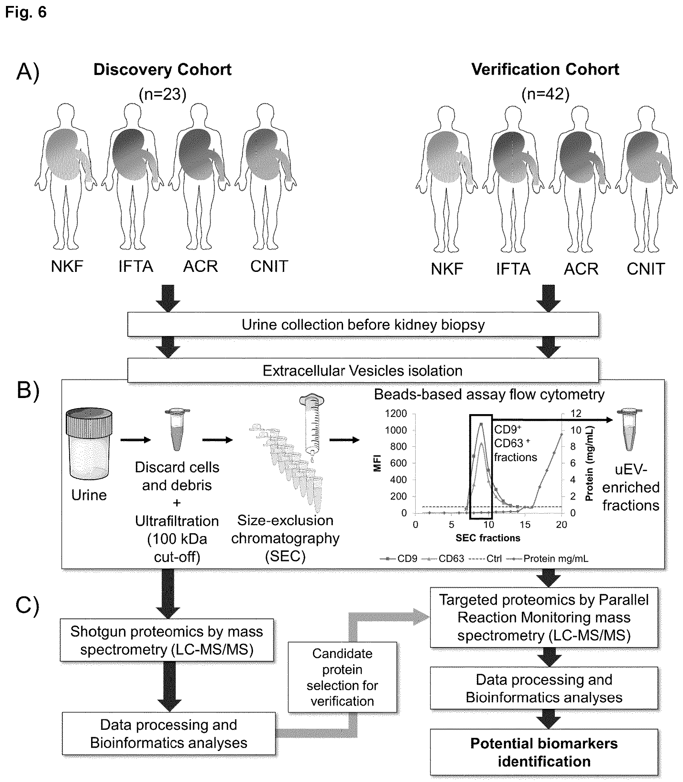

**Fig. 6A****).** Workflow of the study. Urine was collected from patients from the discovery cohort and the verification cohort. Extracellular vesicles were extracted from urine (uEV) by ultrafiltration and size-exclusion chromatography; **B).** The SEC fractions were analysed by beads-based assay flow cytometry to select those with the highest CD9 and CD63 mean fluorescence intensity (MFI); **C).** Samples from the discovery cohort were analysed by shotgun mass-spectrometry. The bioinformatics analyses resulted in the selection of 23 candidate proteins that were analysed by targeted proteomics mass-spectrometry in samples from the verification cohort.

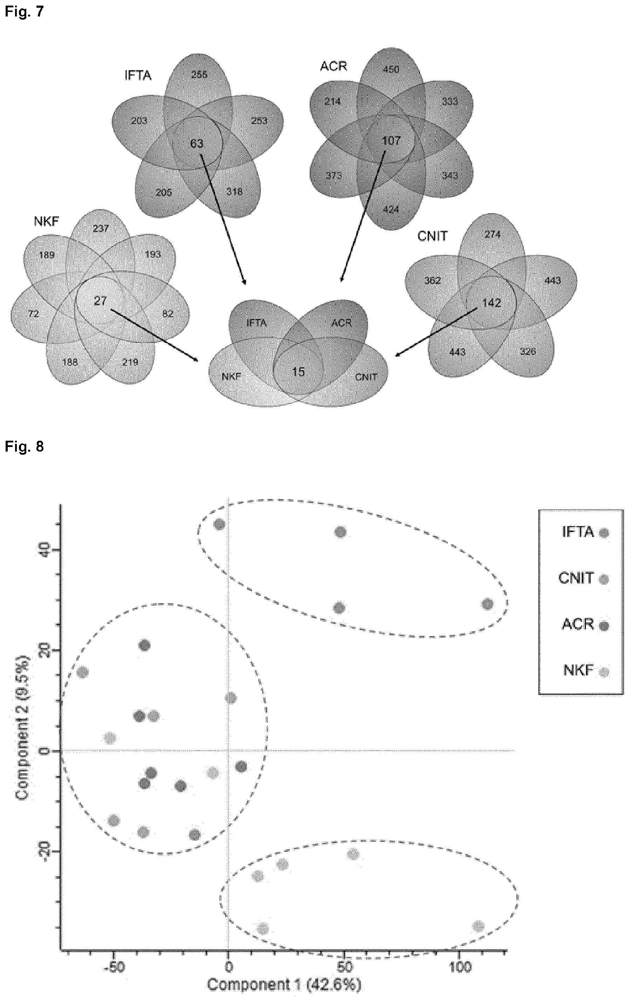

**Fig. 7****.** Venn diagrams showing the number of proteins found in the samples of each group, the number of common proteins between same-group patients, and the number of common proteins between groups. NKF, normal kidney function; IFTA, interstitial fibrosis and tubular atrophy; ACR, acute cellular rejection; CNIT, calcineurin inhibitor toxicity
**Fig. 8****.** Principal component analysis (PCA) that shows the distribution of samples according to Components 1 (x-axis) and 2 (y-axis). Samples were coloured according to their group. Dashed lines circle samples clustering.

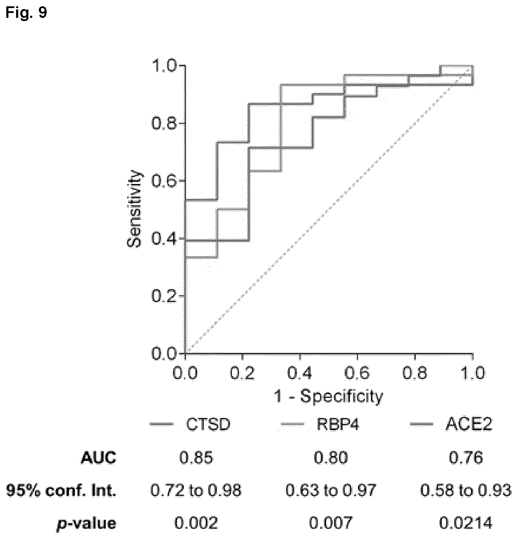

**Fig. 9****.** Receiver operating characteristic (ROC) curves of the three proteins with significantly different expression in NKF compared to pathological samples. AUC, area under the curve; CTSD, Cathepsin-D; RBP4, retinol binding protein-4; ACE2, angiotensin converting enzyme 2; significance p<0.05.

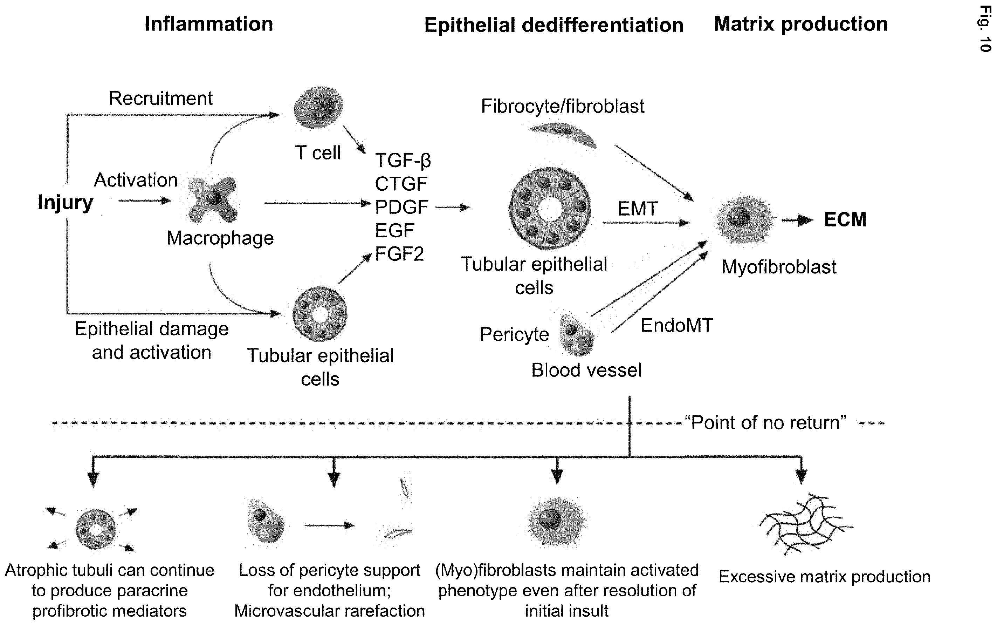

**Fig. 10****.** Simplified diagram of renal fibrogenesis (Fig. 1 from Vanhove, Goldschmeding and Kuypers 2017). Most injurious stimuli result in an inflammatory cascade characterized by recruitment and activation of inflammatory cells, as well as activation of damaged epithelial cells. All of these cell types produce not only proinflammatory but also profibrotic mediators that result in consecutive waves of epithelial dedifferentiation. Resident and recruited mesenchymal cells (fibrocytes, fibroblasts, pericytes) and possibly also epithelial cells (tubular and endothelial) transdifferentiate to become contractile myofibroblasts that produce ECM. When the injury is severe and/or persistent, eventually a "point of no return" may be reached beyond which fibrosis progresses on a local level even after resolution of injury. EndoMT, endothelial-to-mesenchymal transition; FGF, fibroblast growth factor.

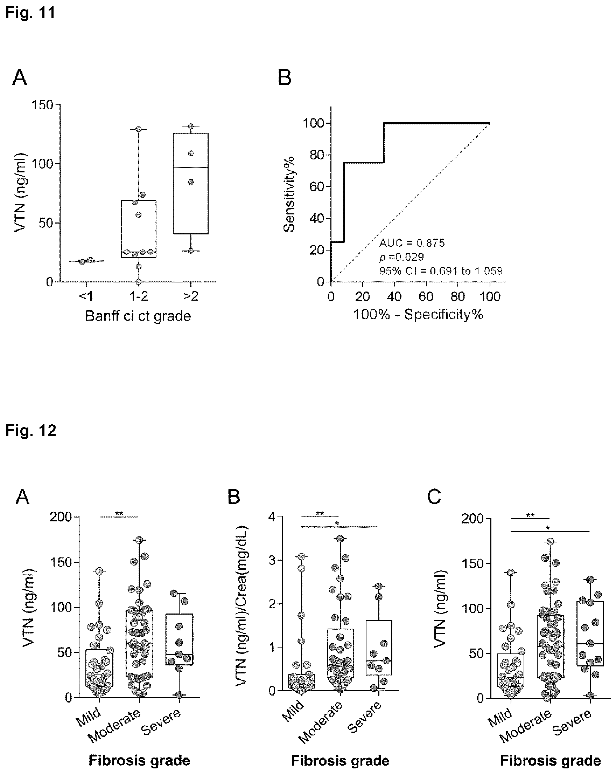

**Fig. 11****.** VTN levels in concentrated urine measured by ELISA related to kidney fibrosis grade. **(A)** VTN concentration in urine was measured by ELISA and the values were stratified regarding the Banff criteria of chronic interstitial and tubular lesions (ci ct) grade. Boxplots show the mean of each group and whiskers show minimum to maximum VTN concentration (ng/mL). **(B)** ROC curve based on ELISA of VTN as a stand-alone biomarker to differentiate Banff ci ct grades ≤ 2 (n=12) from > 2 (n=4). AUC, area under the curve; CI, confidence interval.
**Fig.12****.** Analysis of VTN in concentrated urine by ELISA in non-kidney-transplanted patients with renal alterations. **(A)** Vitronectin concentrations measured by ELISA in urine from non-kidney-transplanted patients with different renal alterations (n=74). **(B)** VTN concentration was corrected by urinary creatinine (n=74). **(C)** VTN concentration measured by ELISA in urine from transplanted and non-transplanted patients (n=97). *, p < 0.05; **, p < 0.01.

### DETAILED DESCRIPTION OF THE INVENTION

### Definitions

The terms "subject", or "individual"' are used herein interchangeably to refer to all the animals classified as mammals and includes but is not limited to domestic and farm animals, primates and humans, for example, human beings, non-human primates, cows, horses, pigs, sheep, goats, dogs, cats, or rodents. Preferably, the subject is a male or female human being of any age or race.

The term "Chronic kidney disease" or "CKD" as use herein may refer to a decrease of renal function associated to a loss of glomerular filtration that progressed over months or years that can eventually lead to end stage renal disease (ESRD). Most individuals may not have any severe symptoms until their kidney disease is advanced. In a particular embodiment, CKD is defined as a persistent abnormality in kidney structure or function (eg, glomerular filtration rate [GFR] <60 mL/min/1.73 m² or albuminuria ≥30 mg per 24 hours) for more than 3 months (Chen, Knicely, and Grams 2019).

The term "diagnosis", as used herein, refers both to the process of attempting to determine and/or identify a possible disease in a subject, i.e. the diagnostic procedure, and to the opinion reached by this process, i.e. the diagnostic opinion. In particular, the diagnosis to determine a disease in a subject, relates to the capacity to identify and classify patients having said disease. A "diagnostic test" could be used to suggest or rule out the disease.

The term "screening" is understood herein as the examination or testing of a group of asymptomatic individuals pertaining to the general population or having one or more risk factors with the objective of discriminating healthy individuals from those who have or are suspected of having a disease. A method of screening is generally used for the "early detection" of a disease. The expression "early detection" refers to detection before the presence of clinical signs.

The term "monitoring" as used herein refers to determining the evolution of the disease and/or the efficacy of a therapy, for example determining whether there is a remission of the disease; or on the contrary whether there is disease progression or a relapse.

The term "Receiver Operating Characteristic (ROC) curves" as used herein refers to a graphical plot that illustrates the performance of a binary classifier system as its discrimination threshold is varied. The curve is created by plotting the true positive rate against the false positive rate at various threshold settings. The true positive rate is also known as sensitivity. The false positive rate is calculated as 1 - specificity. The ROC curve is thus a way of graphically displaying the true positive rate versus the false positive rate (sensitivity vs (1-specificity)) across a range of cut-offs and of selecting the optimal cut-off for clinical use. Accuracy expressed as the area under the ROC curve (AUC) provides a useful parameter for comparing test performance. An AUC approaching 1 indicates that the test is highly sensitive as well as highly specific whereas an AUC approaching 0.5 indicates that the test is neither sensitive nor specific. In general, a test is considered to be a suitable discriminator if the AUC is from 0.6 to 0.75, to have high discrimination capacity if the AUC is from 0.75 to 0.9 and to be an excellent discriminator if the AUC is from 0.9 to 1. For further details see for instance, (Zweig and Campbell 1993; Greiner, Pfeiffer, and Smith 2000).

The term "prognosis" as used herein refers to predicting disease progression or outcome. More specifically, "prognostic markers" may refer to patient characteristics that predict outcome (usually survival) independent of the treatment. The goal of identifying prognostic markers is to define patient subpopulations with significantly different anticipated outcomes, which might benefit from different therapies. Good prognostic patients may not require additional treatment, while poor prognostic patients may derive improved survival benefit from closer clinical follow up or a therapeutic strategy.

The term "treatment" encompasses both a prophylactic or therapeutic treatment. The term "therapeutic treatment" or "therapy" as used herein refers to bringing a body from a pathological state or disease back to its normal, healthy state. The term "prophylactic treatment" as used herein refers to preventing a pathological state. This treatment may be a combined treatment or therapy.

The term "combination therapy" as used throughout the specification, is meant to comprise the administration of the referred therapeutic agents to a subject, in the same or separate pharmaceutical formulations, and at the same time or at different times. If the therapeutic agents are administered at different times they should be administered sufficiently close in time to provide for the combined effect (e.g. potentiating or synergistic response) to occur. The particular combination of therapies to employ in a combination regimen will take into account compatibility of the desired therapeutics and/or procedures and/or the desired therapeutic effect to be achieved. It will be appreciated that the therapies employed may achieve a desired effect for the same disorder, and/or they may achieve different effects (e.g., control of any adverse effects).

The term "therapeutically effective amount" as used herein refers to an amount that is effective, upon single or multiple dose administration to a subject (such as a human patient) in the prophylactic or therapeutic treatment of a disease, disorder or pathological condition.

The term "substantially identical" sequence as used herein refers to a sequence which is at least about 90%, preferably at least about 95%, 96%, 97%, 98%, or 99% identical to a reference sequence. Identity percentage between the two sequences can be determined by any means known in the art, for example the Needleman and Wunsch global alignment algorithm.

The term "reduce or inhibit" as used herein may refer to the ability to cause an overall decrease preferably of 20% or greater, more preferably of 50% or greater, and most preferably of 75%, 85%, 90%, 95%, or greater. Reduce or inhibit can refer for instance, to the biological activity of an active ingredient or a ligand, to the symptoms of the disorder being treated, etc.

The term "affinity reagent" may refer to a ligand (e.g., antibody, peptide, protein, nucleic acid or small molecule) that selectively captures (binds to) a target molecule through specific molecular recognition, typically with a binding affinity in the nanomolar to sub-nanomolar range. For example, the affinity reagent may be an aptamer, antibody or antibody-mimetic.

The term "affinity" as used herein may refer to the equilibrium constant for the dissociation of an antigen with an antigen-binding molecule (KD), and is considered a measure for the binding strength between an antigenic determinant and an antigen-binding site on the antigen -binding molecule: the lesser the value of the KD, the stronger the binding strength between an antigenic determinant and the antigen-binding molecule (alternatively, the affinity can also be expressed as the association constant (KA), which is 1/KD). It will be clear to the skilled person that the dissociation constant may be the actual or apparent dissociation constant.

The term "aptamer" or "nucleic acid aptamer" as used herein may refer to an isolated or purified single-stranded nucleic acid (RNA or DNA) that binds with high specificity and affinity to a target through interactions other than Watson-Crick base pairing. An aptamer has a three-dimensional structure that provides chemical contacts to specifically bind to a target. Unlike traditional nucleic acid binding, aptamer binding is not dependent upon a conserved linear base sequence, but rather a particular secondary or tertiary structure. That is, the nucleic acid sequences of aptamers are non-coding sequences. Any coding potential that an aptamer may possess is entirely fortuitous and plays no role whatsoever in the binding of an aptamer to a target. A typical minimized aptamer is 5-15 kDa in size (15-45 nucleotides), binds to a target with nanomolar to sub-nanomolar affinity, and discriminates against closely related targets (e.g., aptamers will typically not bind to other proteins from the same gene or functional family).

The term "antibody" as used herein may refer to an immunoglobulin or an antigen-binding fragment thereof. Unless otherwise specified, the term includes, but is not limited to, polyclonal, monoclonal, monospecific, multispecific, humanized, human, chimeric, synthetic, recombinant, hybrid, mutated, grafted, and *in vitro* generated antibodies. The antibody can include a constant region, or a portion thereof, such as the kappa, lambda, alpha, gamma, delta, epsilon and mu constant region genes. For example, heavy chain constant regions of the various isotypes can be used, including: IgG₁, IgG₂, IgG₃, IgG₄, IgM, IgA₁, IgA₂, IgD, and IgE. By way of example, the light chain constant region can be kappa or lambda. In certain embodiments, the term "antibody" may also refer to antibody derivatives, such as antibody-based fusion proteins or antibodies further modified to contain additional non-proteinaceous moieties, such as water-soluble polymers, e.g. polyethylene glycol (PEG).

The terms "antigen-binding domain" and "antigen-binding fragment" refer to a part of an antibody molecule that comprises amino acids responsible for the specific binding between antibody and antigen. For certain antigens, the antigen-binding domain or antigen-binding fragment may only bind to a part of the antigen. The part of the antigen that is specifically recognized and bound by the antibody is referred to as the "epitope" or "antigenic determinant." Antigen-binding domains and antigen-binding fragments include Fab; a F(ab')₂ fragment (a bivalent fragment having two Fab fragments linked by a disulfide bridge at the hinge region); a Fv fragment; a single chain Fv fragment (scFv); a Fd fragment (having the two V_{H} and C_{H}1 domains); single domain antibodies (sdAbs; consisting of a single V_{H} domain), and other antibody fragments that retain antigen-binding function. The Fab fragment has V_{H}-C_{H}1 and V_{L}-C_{L} domains covalently linked by a disulfide bond between the constant regions. The Fᵥ fragment is smaller and has V_{H} and V_{L} domains non-covalently linked. The scFᵥ contains a flexible polypeptide that links (1) the C-terminus of V_{H} to the N-terminus of V_{L}, or (2) the C-terminus of V_{L} to the N-terminus of V_{H}. The sdAbs include heavy chain antibodies naturally devoid of light chains and single-domain antibodies derived from conventional four chain antibodies. These antigen-binding domains and fragments are obtained using conventional techniques known to those with skill in the art and are evaluated for function in the same manner as are intact immunoglobulins.

The term "recombinant antibody" as used herein refers to an antibody produced or expressed using a recombinant expression vector, where the expression vector comprises a nucleic acid encoding the recombinant antibody, such that introduction of the expression vector into an appropriate host cell results in the production or expression of the recombinant antibody. Recombinant antibodies may be chimeric or humanized antibodies, mono- or multi-specific antibodies.

The term "an antibody mimetic" (AbM) as used herein refers to single-domain scaffolds, which have been engineered to bind therapeutic targets with affinity and specificity that match that of natural antibodies. Antibody mimetics have been developed utilizing an immunoglobulin-like fold, for example, fibronectin type III, NCAM and CTLA-4. Other mimetics scaffolds bearing no similarity to immunoglobulin folds have also been obtained. Non-limiting examples of said scaffolds are DARPins, anticalins, affibodies, adnectins, fynomers, etc. (see for instance, (Weidle et al. 2013; Löfblom, Frejd, and Ståhl 2011; Banta, Dooley, and Shur 2013)).

The term "marker" or "biomarker" as used herein refers to markers of disease, prognostic or predictive markers which are typically substances found in a bodily sample that can be easily measured. Said bodily sample can be for instance a blood, plasma or feces sample. The term biomarker encompasses biophysical and biochemical determinations, including genetic and serological markers.

The term "proteotypic peptide" as used herein refers to peptides that uniquely represent targeted proteins or protein isoform (Keerthikumar and Mathivanan 2017).

The term "kit" or "testing kit" denotes combinations of reagents and adjuvants required for an analysis. Although a test kit consists in most cases of several units, one-piece analysis elements are also available, which must likewise be regarded as testing kits.

### Detailed description of the invention

### In vitro methods of the invention

VTN is a cell adhesion and spreading factor found in serum and tissues. It has been described to interact with glycosaminoglycans and proteoglycans. It is recognized by certain members of the integrin family and serves as a cell-to-substrate adhesion molecule. The canonical sequence of human VTN is shown below and is referred as SEQ ID NO:5 (UniProtKB Accession Number P04004 -1, sequence version 1 of October 23, 1986) with a length of 478 amino acids and a mass of 54,306 (Da). The sequence in SEQ ID NO:5 may further processed into a mature form. VTN may also be subject to post-translational modifications (e.g., sulfation, phosphorylation, N- and/or O-glycosylation).

MAPLRPLLILALLAWVALADQESCKGRCTEGFNVDKKCQCDELCSYYQSCCTDYTAECKP
QVTRGDVFTMPEDEYTVYDDGEEKNNATVHEQVGGPSLTSDLQAQSKGNPEQTPVLKPEE
EAPAPEVGASKPEGIDSRPETLHPGRPQPPAEEELCSGKPFDAFTDLKNGSLFAFRGQYC
YELDEKAVRPGYPKLIRDVWGIEGPIDAAFTRINCQGKTYLFKGSQYWRFEDGVLDPDYP
RNISDGFDGIPDNVDAALALPAHSYSGRERVYFFKGKQYWEYQFQHQPSQEECEGSSLSA
VFEHFAMMQRDSWEDIFELLFWGRTSAGTRQPQFISRDWHGVPGQVDAAMAGRIYISGMA
PRPSLAKKQRFRHRNRKGYRSQRGHSRGRNQNSRRPSRATWLSLFSSEESNLGANNYDDY
RMDWLVPATCEPIQSVFFFSGDKYYRVNLRTRRVDTVDPPYPRSIAQYWLGCPAPGHL

The term "VTN" as used herein refers to human VTN protein with SEQ ID NO: 5 and to sequences substantially identical thereto. Preferably, said sequence is SEQ ID NO: 5.

### Method for the screening, diagnosis or monitoring of renal fibrosis

The inventors have also determined the value of vitronectin (VTN) as marker of the degree of kidney fibrosis, which is a disorder typically associated to IFTA but which may also occur in other kidney diseases (e.g. CNIT).

Indeed, renal Fibrosis has been described as the common pathway for progression of chronic kidney disease (CKD) to end stage of renal disease (Vivekanand Jha et al, Lancet 2013. Doi: 10.1016/S0140-6736(13)60687-X). It has been described that the degree of renal fibrosis correlates with kidney function and CKD stages. The number of people suffering CKD is enormously higher compared to kidney transplanted patients - around 25,000,000 people both in Europe and in the United States. Fibrosis eventually develops during the progression of CKD and it is the best histologic predictor of renal function decline (Humphreys 2018). Patients suffering from chronic kidney disease may have *inter alia* underlying glomerulosclerosis, amyloidosis, diabetic nephropathy or IgA nephropathy.

The inability to detect kidney fibrosis early, before irreversible ECM accumulation, limits the development of therapeutics. As mentioned above, there is a need to identify those patients with a low-moderate degree of fibrosis which would likely benefit from the treatment with antifibrotic agents. (Vanhove, Goldschmeding, and Kuypers 2017) illustrate in Fig. 1 (included as Fig.10) the process of renal fibrogenesis. They describe that there is a *"point* of *no return"* of structural injury beyond which fibrosis progresses at local level even after resolution of injury. Although fibrosis does not spread throughout the graft, each allograft has a limited reserve capacity beyond which structural and functional deterioration will progress in the absence of persistent injury. This occurs if nephron loss reaches a critical point, after which compensatory hyperfiltration results in hypertensive damage to the remaining glomeruli and proteinuria can result in sustained tubular injury.

Thus, another aspect of the invention relates to the *in vitro* use of VTN protein levels as kidney fibrosis biomarker. In a related aspect, the invention pertains to a method for the screening, diagnosis or monitoring of kidney fibrosis in a subject, wherein said method comprises or consists of:
a. determining vitronectin (VTN) protein levels in a urine sample from said subject; and
b. determining whether the VTN protein levels in the sample are increased with respect to a reference value;
wherein VTN protein levels in a) increased with respect to the reference value is indicative of fibrosis.

The inventors surprisingly found that VTN was associated to the degree of kidney fibrosis. For instance, VTN can be useful for discriminating in a statistically significant manner low or mild (<1 cict BANNF criteria) and moderate (1-2 cict BANNF criteria) from severe (>2 cict BANNF criteria) fibrosis as shown in Fig. 5 or Fig. 11. A person skilled in the art will know how to determine the most appropriate reference value depending on whether he wants to discriminate low-moderate fibrosis from severe fibrosis (e.g., to identify those patients likely to respond to antifibrotic treatment) or he desires to select those patients with moderate-severe fibrosis who might have a worst prognosis. The terms low or mild fibrosis are used indistinctively herein.

In some embodiments, levels of fibrosis above the reference value are indicative of severe fibrosis. For instance, as referred above, the reference value may be selected to discriminate low (mild) -moderate fibrosis from severe fibrosis and increased VTN protein levels in a) with regard to the reference value is indicative of severe kidney fibrosis; and decreased VTN protein levels in a) with regard to the reference value is indicative of low (mild) or moderate kidney fibrosis.

Fibrosis may be measured by any method known in the art. For instance, it can be assessed using the Banff scoring system (Kim Solez et al. 1993; revisited: Haas et al. 2018), by computerized quantification of interstitial fibrosis (Servais et al. 2009), or by histopathological analysis further to a trichrome staining protocol, such as with hematoxylin and eosin, PAS and Silver Methenamine (Farrris et la. 2011). Preferably, severe fibrosis corresponds to a mean score of Banff criteria chronic interstitial (ci) lesions and chronic tubular (ct) lesions (BANF ci ct) in a renal biopsy of more than 2, wherein each of ci and ct lesions are parameters which can have values selected from 0, 1, 2 or 3, thus including subjects with mean values of 2.5 and 3. More preferably, it corresponds to a mean score of Banff ci and ct lesions in a renal biopsy of 3. In some embodiments, VTN protein levels in a) increased with respect to the reference value is indicative of IFTA with severe fibrosis.

In other embodiments, levels of fibrosis above the reference value are indicative of moderate-severe fibrosis. For instance, as referred above, the reference value may be selected to discriminate moderate-severe fibrosis from low fibrosis and increased VTN protein levels in a) with regard to the reference value is indicative of moderate-severe kidney fibrosis; and decreased VTN protein levels in a) with regard to the reference value is indicative of low (mild) kidney fibrosis.

Preferably, moderate to severe fibrosis corresponds to Banff ci ct above 1, more preferably above 1.5.

As mentioned above, the inventors surprisingly found that VTN levels enabled to distinguish two patient subgroups according to their degree of fibrosis within those patients classified by a pathologist as having IFTA. Thus, a related aspect of the present invention concerns an *in vitro* method for classifying a subject according to its degree of kidney fibrosis, said method comprising determining whether VTN protein levels in a biological sample (e.g. a urine sample) from said subject are increased with respect to a reference value, wherein increased VTN protein levels in said biological sample with respect to a reference value is indicative of severe or moderate-severe kidney fibrosis.

Particular embodiments and preferred features are as described herein for other aspects of the invention.

In a further related aspect, the present invention provides a method for determining the degree of kidney fibrosis in a subject, wherein said method comprises or consists of:
a. determining vitronectin (VTN) protein levels in a urine sample from said subject; and
b. determining whether the VTN protein levels in the sample are increased with respect to a reference value;
wherein increased VTN protein levels in a) with regard to the reference value is indicative of severe or moderate-severe kidney fibrosis.

Particular embodiments and preferred features are as described herein for other aspects of the invention.

In some embodiments, the methods described herein may enable discriminating and classifying subjects in two distinct degrees of kidney fibrosis according to VTN levels, such as (i) low-moderate from severe or (ii) low from moderate-severe. In other embodiments, it may enable discriminating and classify subjects in three distinct degrees of kidney fibrosis, namely distinguishing between low, moderate and severe kidney fibrosis.

The methods described herein may be useful for the early detection of patients presenting renal fibrosis, such as a severe or moderate-severe degree of kidney fibrosis. These patients can be candidates for further exploratory tests, such as a renal biopsy which is the current gold standard. Accordingly, in some embodiments, a method for the screening, diagnosis, or monitoring of kidney fibrosis, of determining the degree of kidney fibrosis or of classifying a subject according to the degree of renal fibrosis as described herein, further comprises conducting an exploratory test, such as a renal biopsy, in those subjects found to have a severe or moderate-severe degree of kidney fibrosis.

In some embodiments, said subject has chronic kidney disease. In other embodiments, said subject is a kidney transplanted patient. For instance, a subject suffering from chronic kidney disease which was subject to renal replacement therapy. In some other embodiments, optionally in combination with any of the embodiments described herein, the subject according to the methods of the invention has IFTA.

In a further aspect, the invention relates to a method for determining the prognosis of a subject on the basis of its degree of kidney fibrosis, preferably a kidney transplanted patient, wherein said method comprises or consists of:
a. determining vitronectin (VTN) protein levels in a urine sample from said subject; and
b. determining whether the VTN protein levels in the sample are increased with respect to a reference value;
wherein VTN protein levels in a) increased with respect to the reference value is indicative of severe or moderate-severe kidney fibrosis and poor prognosis, for instance it predicts future functional decline and in transplanted patients a higher risk of graft-loss.

As described herein, the degree of fibrosis has been associated with prognosis, those patients with severe fibrosis are those with worst predicted outcome (Vanhove, Goldschmeding, and Kuypers 2017; Serón et al. 2002; Pape et al. 2003; Fernando G. Cosio, Grande, Wadei, et al. 2005; F. G. Cosio et al. 2016).

**Step (a)** of the method under these aspects of the invention comprises or consists of determining in said biological sample the protein levels of VTN. Combinations with other biomarkers may be as described herein above.

The methods of the invention can be applied to any type of biological sample isolated from a patient, such as a biopsy sample, tissue, cell or fluid. Preferably, said biological sample is a biological fluid, including urine, serum, blood, saliva, semen, sputum, cerebral spinal fluid (CSF), tears, mucus, sweat, milk, brain extracts and the like. Preferably, said biological sample is a urine sample.

These types of samples are routinely used in the clinical practice and a person skilled in the art will know how to identify the most appropriate means for their obtaining and preservation. Once a sample has been obtained, it may be used fresh, it may be frozen or preserved using appropriate means.

In some embodiments, said sample is a extracellular vesicles (EVs)-enriched sample, preferably a urine EVs-enriched sample. Suitable methods for EV isolation and/or concentration are well known in the art and include ultracentrifugation, density gradient, ultrafiltration, asymmetrical flow field-flow fractionation, precipitation with precipitating agents and immunoaffinity, size-exclusion chromatography, ion-exchange chromatography, tangential filtration, and any other method suitable to enrich or isolate EV's, as well as combinations thereof.

In a particular embodiment, said urine extracellular vesicles (EVs)-enriched sample is obtained by concentration of the urine sample. For instance, said urine EVs-enriched sample may in some embodiments be obtained by a process comprising or consisting of urine centrifugation and selection of the supernatant, and subsequent concentration by ultrafiltration. A filter cut-off of more than 10 kDa can be used, preferably of more than 20 kDa, 30 kDa, 40 kDa, 50 kDa, 60 kDa, 70 kDa, 80 kDa, 90 kDa, 100 kDa, 150 kDa or 200 kDa. In preferred embodiments, the filter cut-off is of 20 kDa to 150 kDa, more preferably of 50 kDa to 100 kDa, even more preferably of 50 kDa.

Alternatively, said urine EVs-enriched sample has been isolated from urine by a process comprising size-exclusion chromatography (SEC) which may optionally comprise the steps described herein above for sample preparation. In a particular embodiment, said urine EVs-enriched sample has been isolated from urine by a process comprising or consisting of urine concentration and EV isolation by size-exclusion chromatography (SEC). A precise protocol for isolating EV's by SEC is described in the Examples and in Monguió-Tortajada, Gálvez-Montón, et al. 2019.

SEC, also known as gel filtration, separates molecules by differences in size as they pass through a SEC resin packed in a column. SEC resins consist of a porous matrix of spherical particles that lack reactivity and adsorptive properties. After sample has been applied, molecules larger than the pores are unable to diffuse into the beads, so they elute first. Molecules that range in size between the very big and very small can penetrate the pores to varying degrees based on their size. If a molecule is smaller than the smallest of the pores in the resin, it will be able to enter the total pore volume. Molecules that enter the total pore volume are eluted last. Samples are eluted isocratically so there is no need to use different buffers during the separation. For instance, a SEC resin that may be used in the method of the invention has a particle size of 50 to 250 µm, preferably of 60 to 200 µm. The matrix pore size can be of 50 to 100 nm, preferably of about 75 nm. In preferred embodiments, said SEC resin has a particle size of 60 to 200 µm and a matrix pore size of 75 nm, such as sepharose CL-2B.

For instance, said urine EVs-enriched sample is obtained from urine by a process comprising or consisting of the following steps:
a. Subjecting the urine to ultracentrifugation and selecting the supernatant;
b. Optionally, concentrating the supernatant as described herein above;
c. Loading the supernatant or the concentrate to a SEC column,
d. Collecting eluted EV-enriched fractions.

EV-enriched fractions may be identified as those eluted fractions presenting the highest expression of EV markers. Typical EV markers detected in urine are CD9 and CD63, but other known EV markers, for instance ezrin can also be detected.

EV-enriched samples may be subject to lysis. Methods for EV lysis are well known in the art and include but are not limited to detergent extraction, freeze-thawing serial treatment and sonication. In some embodiments, an EV lysis step is not conducted (e.g. the detected protein is an EV surface protein).

Suitable methods for determining the levels of a given protein include, without limitation, those described herein below. Preferred methods for determining the protein expression levels in the methods of the present invention are immunoassays. Various types of immunoassays are known to one skilled in the art for the quantitation of proteins of interest. These methods are based on the use of affinity reagents, which may be any antibody or ligand specifically binding to the target protein or to a fragment thereof, wherein said affinity reagent is preferably labeled. Illustrative, but non-exclusive, examples of labels that can be used include radioactive isotopes, enzymes, fluorophores, chemoluminescent reagents, enzyme cofactors or substrates, enzyme inhibitors, particles, dyes, etc.

Affinity reagents may be any antibody or ligand specifically binding to the target protein or to a fragment thereof. Affinity ligands may include proteins, peptides, peptide aptamers, affimers and other target specific protein scaffolds, like antibody-mimetics.

Specific antibodies against the protein markers used in the methods of the invention may be produced for example by immunizing a host (e.g. mice or rabbit) with the target protein or a fragment thereof. Likewise, peptides specific against the protein markers used in the methods of the invention may be produced by screening synthetic peptide libraries.

Western blot or immunoblotting techniques allow comparison of relative abundance of proteins separated by an electrophoretic gel (e.g., native proteins by 3-D structure or denatured proteins by the length of the polypeptide). Immunoblotting techniques use antibodies (or other specific ligands in related techniques) to identify target proteins among a number of unrelated protein species. They involve identification of protein target via antigen-antibody (or protein-ligand) specific reactions. Proteins are typically separated by electrophoresis and transferred onto a sheet of polymeric material (generally nitrocellulose, nylon, or polyvinylidene difluoride). Dot and slot blots are simplified procedures in which protein samples are not separated by electrophoresis but immobilized directly onto a membrane.

Traditionally, quantification of proteins in solution has been carried out by immunoassays on a solid support. Said immunoassay may be for example an enzyme-linked immunosorbent assay (ELISA), a fluorescent immunosorbent assay (FIA), a chemiluminescence immunoassay (CIA), or a radioimmunoassay (RIA), an immunoradiometric assay (IRMA), an enzyme multiplied immunoassay, a solid phase radioimmunoassay (SPROA), a fluorescence polarization (FP) assay, a fluorescence resonance energy transfer (FRET) assay, a time-resolved fluorescence resonance energy transfer (TR-FRET) assay, a surface plasmon resonance (SPR) assay, magnetic beads-based immunoassay (MBI), an electro-chemiluminescent immunoassay (ECL). Multiplex and any next generation versions of any of the above, such as bead-based flow-cytometry immunoassays (e.g., based on the Luminex xMAP technology) are specifically encompassed. In a particular embodiment, said immunoassay is an ELISA assay or any multiplex version thereof.

Other methods that can be used for quantification of proteins in solution are techniques based on mass spectrometry (MS) such as liquid chromatography coupled to mass spectrometry (LC / MS), described for example in US2010/0173786, or tandem LC-MS / MS (WO2012/155019, US2011/0039287; (Rauh 2012)) and multiplex versions of the above techniques, as well as the next generation of such techniques and combinations thereof.

In other embodiments, determination of whether VTN protein levels are increased with respect to a reference value is conducted by lateral flow tests, also known as Lateral Flow Immunochromatography (LFIC) tests or just Lateral Flow Immunoassays (LFI). These are simple small devices intended to detect a target analyte in a given sample. These tests are simple, fast, and cheap and do not require specialized and costly equipment nor qualified personnel. In the beginning, LFIC tests were exclusively used in medical diagnostics (either for home testing, point of care testing, or laboratory use) but their presence in other areas are now very common. The first application was the well-known home pregnancy test. Although there are several commercially available semi-quantitative tests, LFIC tests are mainly qualitative tests and they just give a positive or negative result based on the presence or absence of the target analyte at a specific concentration.

The technology is based on a series of capillary beds with porous materials that has the capacity to transport liquids spontaneously. When the target analyte is big (e.g. a protein) a sandwich format is commonly used. Usually, the first element (the conjugate pad) has stored the so-called colloids, a dried format of bioactive particles (mainly gold nanoparticles or latex microparticles bound to a specific antibody against the target analyte) in a salt-sugar matrix. This conjugate pad usually acts also as the sample pad and it contains everything to facilitate the recognition of the target antigen by the colloid. Once the conjugate pad is soaked by the sample, the fluid dissolves the salt-sugar matrix and the colloids. Therefore, the sample and the colloid mix while they flow through the porous structure. In this way, the analyte binds to the colloid and the complex is transported into the nitrocellulose membrane. This material has one specific area called test line where a third molecule (usually another different antibody against the target analyte) has been immobilized. When the complex (colloid-antigen) reaches the test line it gets bound to this antibody and after a while, when more and more fluid has flown, colloids accumulate, and the test line acquires color. Typically, there is also a control line (after the test line) that captures the colloids that have not bound to the test line. In the control line, a fourth molecule (could be an antibody against the Fc fraction of the first antibody) is adsorbed and its color acquisition ensures the test functionality. After passing these reaction zones the fluid enters the final porous material, the wick or sink that simply acts as a waste container.

VTN protein levels can be absolute or relative. For instance, normalization can be conducted by expressing the levels of the target protein relative to the levels of another protein, such as housekeeping proteins (e.g. β-Actin, GAPDH, HPRT1, or RPLP1) or creatinine. In other instances, normalization can be conducted by total protein normalization.

Normalization of experimental protein biomarkers in urine by creatinine levels is commonly used because creatinine is considered to be secreted at a more or less constant rate in urine. However, creatinine excretion suffers large individuals variations and is profoundly affected by renal pathologies (Weaver et al. 2016; Greenblatt et al. 1976). In uEV, normalization by urinary creatinine has been shown to lead to bias in results interpretation (Gheinani et al. 2018).

As shown in Example 5, the normalization of creatinine did not substantially modify the results. In particular embodiments, the methods of the invention do not comprise normalization of the VTN levels by creatinine.

The methods described herein comprise comparing the protein levels or score in the subject sample with a reference value. The term "reference value", as used herein, relates to a predetermined criterium used as a reference for evaluating the values or data obtained from the samples collected from a subject. This "reference value" may also be referred as "cut-off value" or "threshold value".

The term "reference value" may refer to a value determined in one or more samples, generally mean values of preferably a statistically significant number of samples, obtained from a reference or control population. Determination of the "reference value" is carried out under similar or identical conditions as the biological sample of the tested subject. Typically, said reference samples are from the same type of biological sample, e.g., the same tissue or cell type and have undergone the same procedures for their obtaining and preservation. Alternatively; said "reference values" are pre-established values, generally mean values, in the control or reference population.

The reference value can be an absolute value, a relative value, a value that has an upper or a lower limit, a range of values, an average value, a median value, a mean value, a z-score (e.g. mean value + or - 1, 2 or 3 standard deviation (SD)), a tertile value, or a value as compared to a particular control or baseline value. In a particular embodiment, optionally in combination with one or more of the embodiments or features described above or below, said reference value is the mean value or the tertile value.

In addition, it is further noted that a variety of statistical and mathematical methods for establishing the threshold or cut-off level of expression are known in the prior art. A threshold or cut-off expression level for a particular biomarker may be selected, for example, based on data from Receiver Operating Characteristic (ROC) plots. One of skill in the art will appreciate that these threshold or cut-off expression levels can be varied, for example, by moving along the ROC plot for a particular biomarker or combinations thereof, to obtain different values for sensitivity or specificity thereby affecting overall assay performance.

Sensitivity, specificity, and/or accuracy are parameters typically used to describe the validity or performance of a test. In particular, they are used to quantify how good and reliable the discrimination method is. A test is usually calibrated in terms of the desired specificity and sensitivity according to the target use of the test in clinical practice. High sensitivity corresponds to high negative predictive value and is considered generally a desired property for a "rule out" test, such as a screening test which typically will be followed by a confirmatory test. High specificity corresponds to high positive predictive value and is considered generally a desired property for a "rule in" test, such as a companion diagnostic test.

In preferred embodiments, the methods of the invention have sensitivity, specificity and/or accuracy values of at least about 60 %, preferably of at least about 70 %, and can be, for example, at least 75 %, at least 80 %, at least 85 %, at least 90 %, at least 95 %, at least 96 %, at least 97 %, at least 98 %, at least 99 % or 100% in at least 60 % of the group or population assayed, or preferably in at least 65 %, 70 %, 75 %, 80 %, 85 %, 90 %, 95 % or 100 % of the group or population assayed.

In the methods of the invention, the protein levels or the score are considered "decreased" when said value is lower than a reference value. Preferably, the value is considered to be lower than a reference value when it is at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more lower than the reference value.

Likewise, in the context of the methods of the invention, the protein levels or the score is considered "increased" when said value is higher than a reference value. Preferably, the value is considered to be higher than a reference value when it is at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 100%, at least 110%, at least 120%, at least 130%, at least 140%, at least 150%, or more higher than a reference value.

Alternatively, or in addition, subjects having more than about 1.1,1.2, 1.3, 1.4, 1.5, 2, 3, 4, 5, 6, 7, 8, 9, 10, 15 or 20 fold levels deviation (i.e., increase or decrease) than the reference value as described herein.

The method of the invention, as it is understood by a person skilled in the art, does not claim to be correct in 100% of the analyzed samples. However, it requires that a statistically significant amount of the analyzed samples are classified correctly. The amount that is statistically significant can be established by a person skilled in the art by means of using different statistical significance measures obtained by statistical tests; illustrative, non-limiting examples of said statistical significance measures include determining confidence intervals, determining the p-value, etc. Preferred confidence intervals are at least 90%, at least 95%, at least 97%, at least 98%, at least 99%. The p-values are, preferably less than 0.1, less than 0.05, less than 0.01, less than 0.005 or less than 0.0001. The teachings of the present invention preferably allow correctly classifying at least 60%, at least 70%, at least 80%, or at least 90% of the subjects of a determining group or population analyzed.

It is further noted that the accuracy of the method of the invention can be further increased by additionally considering other gene markers, biochemical parameters and/or clinical characteristics of the patients (e.g. age, sex, tobacco and/or other risk factors). Determination of these other markers, parameters and/or characteristics can be sequential or simultaneous to any or all of the steps described herein above.

The subject in the methods of the invention is preferably a human subject. Said subject can be alive or a cadaveric subject.

The methods described herein or any of the steps thereof might be implemented by a computer. In a particular embodiment, optionally in combination with any of the features or embodiments as described herein, the score obtained by mathematical combination of the determined protein levels is calculated using a computer; and/or the comparison between the values in the subject sample and the reference value is conducted using a computer.

Therefore, a further aspect of the invention refers to a computer implemented method as defined in the claims.

It is noted that any computer program capable of implementing any of the methods of the present invention or used to implement any of these methods or any combination thereof, is also described herein.

This computer program is typically directly loadable into the internal memory of a digital computer, comprising software code portions for performing the steps of comparing the values from the one or more biological samples of a subject with a reference value and determining renal damage or pathological phenotype, acute renal rejection, kidney fibrosis or poor prognosis as described herein above, when said product is run on a computer.

It is also noted that any device or apparatus comprising means for carrying out the steps of any of the methods of the present invention or any combination thereof, or carrying a computer program capable of, or for implementing any of the methods of the present invention or any combination thereof, is included as forming part of the present specification.

The methods of the invention may also comprise the storing of the method results in a data carrier, preferably wherein said data carrier is a computer readable medium. It is further described a computer-readable storage medium having stored thereon a computer program as described herein or the results of any of the methods of the invention.

As used herein, "a computer readable medium" can be any apparatus that may include, store, communicate, propagate, or transport the results of the determination of the method of the invention. The medium can be an electronic, magnetic, optical, electromagnetic, infrared, or semiconductor system (or apparatus or device) or a propagation medium.

### Methods of subject selection and treatment of the invention

In an additional aspect, the present invention concerns a method for selecting a subject which would likely benefit from the administration of an agent suitable for the preventive and/or therapeutic treatment of fibrosis as described herein, wherein said patient is selected for presenting low or moderate fibrosis, according to the method as described herein above. A treatment or agent for the preventive and/or therapeutic treatment of fibrosis can be generally referred as antifibrotic treatment or agent.

It is described herein a method for treating a subject with kidney fibrosis, preferably low or moderate fibrosis, by administering a therapeutically effective amount of an agent suitable for the preventive and/or therapeutic treatment of fibrosis, wherein said patient has been determined to have kidney fibrosis according to a method as described herein above.

This treatment may be any known treatment suitable for the preventive and/or therapeutic treatment of fibrosis. A patient suspected of having fibrosis or IFTA can be treated with corticoid drugs, such as described herein above, and/or antihypertensive drugs, such as angiotensin converting enzyme inhibitors (ACEls), angiotensin II receptor blockers (ARBs), and/or calcium antagonists, since these have been described to be nephroprotective. In addition, in kidney transplanted patients it may be reduced/avoided the use of anticalcineurinic drugs, such as Cyclosporin A (CyA) and tacrolimus (TAC).

Alternatively, or in addition, other agents can also be used, such as those described in Table 1 of Vanhove, Goldschmeding, and Kuypers 2017 (reproduced below) including any combination thereof. These include anti-tumor growth factor β (TGFβ) and anti-connective tissue growth factor (CTGF) agents *interalia.*

**Table 1. Studies of renal antifibrotic interventions in humans**

| **Setting** | **Molecular** target | **Intervention** | **Study type** | **Results** | **Ref** |
|---|---|---|---|---|---|
| **FSGS** | TGF-β | Pirfenidone | Open label, nonrandomized | 25% slower decline in eGFR | 190 |
| | TGF-β | Fresolimumab (antibody) | Phase I | Well tolerated. No studies regarding efficacy | 191 |
| | CTGF | FG-3019 (antibody) | Phase I | Early termination | 192 |
| **Diabetic nephropathy** | TGF-β | Pirfenidone | Phase III | Improvement in eGFR after 1 year (only in 1200 mg/d arm) vs decrease in placebo group. No difference in proteinuria. | 193 |
| | TGF-β | LY2382770 (antibody) | Phase II | Terminated early: lack of efficacy on eGFR | 194 |
| | CTGF | FG-3019 (antibody) | Phase I | Reduction in albuminuria | 195 |
| | CTGF | FG-3019 (antibody) | Phase II | Early termination | 196 |
| | Oxidative stress | GKT137831(NOX1/4 inhibitor) | Phase II | Completed, not yet published | 197 |
| | Oxidative stress | Pyridoxamine (inhibits oxidative stress and AGE formation) | Phase III | Ongoing | 198 |
| | Chemotaxis | CCX140-B (CCR2 blocker) | Phase II | Reduction in albuminuria | 199 |
| | Chemotaxis | Bindarit (inhibitor of CCL2, -7, and -8) | Phase II | Completed, not yet published | 200 |
| | Prostaglandins | Pentoxifylline | Multiple studies | Modest reductions in albuminuria and rate of eGFR decline. RCT powered to detect difference in serum creatinine doubling or ESRD occurrence is ongoing | 202, 203 |
| | Prostaglandins | PF-00489791 (PDE5 inhibitor) | Phase III | Reduction in albuminuria | 204 |
| | Prostaglandins | CTP-499 (multispecific PDE inhibitor) | Phase I | Well tolerated. No data on efficacy. | 205 |
| | Prostaglandins | Beraprost (oral prostacyclin analogue) | Phase II | No difference in slope of 1/SCr after 28 weeks | 206 |
| | Endothelin | Avosentan (endothelin A receptor antagonist) | Phase III | Early termination: reduction in proteinuria but increased mortality | 207 |
| | Endothelin | Atrasentan (endothelin A receptor antagonist) | Phase III | Ongoing | 208 |
| | JAK pathway | Baricitinib (JAK1 and -2 inhibitor) | Phase III | Completed, not yet published | 209 |
| **Prevention** of **cardiac surgery-related acute kidney injury** | BMP | Thr-184 | Phase II | Completed, not yet published | 210 |

| | | | | | |
|---|---|---|---|---|---|
| Pirfenidome has multiple mechanisms, 1 of which is blocking the TGF-β promoter; Pentosifylline is a nonspecific PDE inhibitor, inhibits Smad3/4 and CTGF and has anti-inflammatory properties. AGE, advanced glycation end product; BMP, bone morphogenetic protein; CCR, C-C chemokine receptor type 2; FSGS, focal segmental glomerulosclerosis; JAK, Janus kinase; NOX, NADPH oxidase; PDE: phosphodiesterase. | | | | | |

Inflammasomes are multimeric proteins that promote immune responses and the programmed cell death process known as pyroptosis by the activation of caspase-1 in response to pathogen-associated molecular patterns (PAMPs) or danger-associated molecular patterns (DAMPs). In preferred embodiments, the method for treating a subject with kidney fibrosis of the invention comprises administering a therapeutically effective amount of inhibitors of the inflammasome optionally in combination with one or more of the agents described herein above.

The priming of the inflammasome is initiated by the recognition of a PAMP or DAMP, which mediates the activation of NF-κB. The activation of NF-κB induces the production of NLRP3 and the generation of pro-IL-1β and pro-IL-18. After deubiquitination and combination with mtDNA, NLRP3 interacts with ASC and caspase-1, forming the inflammasome complex. The inflammasome is activated by the recognition of P2X7R, leading to the cleavage of caspase-1. Active caspase-1 then promotes the secretion of IL-1β and IL-18, which is the key to inducing inflammasome-dependent inflammation (Xu et al. 2019).

The terms "(NLRP3) inflammasome inhibitor" or "inhibitor of the (NLRP3) inflammasome pathway" are used herein indistinctively and refer to an agent inhibiting inflammasome signaling by any means. Illustrative, non-limiting examples of "inflammasomme inhibitors" are those inhibiting NF-κB, NLRP3, caspase-1, IL-1β or IL-18 signaling, for instance at any of the (i) ligand level (e.g., antisense oligonucleotides to prevent the target molecule synthesis) or (ii) the ligand-receptor level (e.g., target molecule-neutralizing affinity ligands [e.g., monoclonal antibodies] and soluble receptors), or (iii) the intracellular level, which includes agents preventing signal transduction. Illustrative non-limiting examples include those described in Shao et al. 2015 and Zahid et al. 2019 Some of these have been highlighted below:
**1. Type I interferon (IFN) and IFN-β.** Inducting phosporylation of STAT1, transcription factor, Inducting IL-10 production.
**2. Autophagy inducer**
   **a. Resveratrol.** Inducing autophagy process, Suppressing mitochondrial damage.
   **b. Arglabin.** Inducing autophagy process, Reducing cholesterol level.
   **c. CB2R agonist.** Inducing autophagy process, Inhibiting priming step of NLRP3 inflammasome activation.
**3. MicroRNA**
   **a. MicroRNA-223.** Suppressing NLRP3 protein expression.
**4. Indirect inhibitors**
   **a. Glyburide.** Inhibits ATP-sensitive K+ channels; downstream of P2X7 resulting in inhibition of ASC aggregation
   **b. 16673-34-0.** Induces NLRP3 conformational changes secondary to its activation or binding to ASC
   **c. JC124.** Blocks the expression of NLRP3, ASC, caspase-1, pro-IL-1β, TNFα and iNOS
   **d. FC11A-2.** Interferes with proximity induced autocleavage of pro-caspase-1, suppresses IL-1β/18 release
**5. Inhibitors for the constituents of NLRP3 inflammasome**
   **a. Parthenolide.** Alkylates cysteine residues in caspase-1 and in ATPase domain of NLRP3, inhibits NLRP3 ATPase activity
   **b. VX-740.** Covalent modification of the catalytic cysteine residue in the active site of caspase-1 resulting in caspase-1 blocking and resultant cleavage of pro-IL-1β/18
   **c. VX-765.** Covalent modification of the catalytic cysteine residue in the active site of caspase-1 resulting in caspase-1 blocking and resultant cleavage of pro-IL-1β/18
   **d. Bay 11-7082.** Alkylates the cysteines in the ATPase domain of NLRP3, inhibits NLRP3 ATPase activity
   **e. β-Hydroxybutyrate (BHB).** Blocking ASC oligomerization, Inhibiting K+/potassium efflux.
**6. Direct inhibitors of NLRP3 protein**
   a. **MCC950.** Blocking apoptosis-associated speck-like protein (ASC) oligomerization, Inhibiting of canonical and non-canonical NLRP3 inflammasome.
   **b. 3,4-Methylenedioxy-β-nitrostyrene (MNS).** Inhibits NLRP3 ATPase activity by cysteine modification, blocks NLRP3 inflammasome activation
   **c. CY-09.** Inhibits NLRP3 ATPase activity, blocks NLRP3 inflammasome activation
   **d. Tranilast.** Binds to NLRP3 NACHT domain to block NLRP3-NLRP3 and NLRP3-ASC interaction
   **e. OLT1177.** Inhibits NLRP3 ATPase activity, blocks NLRP3 inflammasome activation
   **f. Oridonin.** Binds to cysteine 279 of NLRP3 to abolish NLRP3-NEK7 interaction, blocks NLRP3 inflammasome activation
   **g. IFM-2427.** NLRP3 antagonist
   **h. JT-194.** NLRP3 assembly inhibitors
   **i. JT-394.** NLRP3 assembly inhibitors

NLRP3-specific inflammasome inhibitors have been described to attenuate kidney fibrosis in mice and are presently studied as promising treatments for kidney fibrosis (Ludwig-Portugall et al. 2016; Krishnan et al. 2019). In some embodiments, said inhibitors of the inflammasome are NLRP3-specific inflammasome inhibitors.

### Kits and uses of the invention

It is further described herein a kit for determining the levels of one or more of the target markers as described herein in a biological sample as described herein (preferably a urine sample) isolated from a subject. Combinations of markers can be as described above under the methods of the invention. The kit may also contain instructions indicating how the materials within the kit may be used.

The term "kit" or "testing kit" denotes combinations of reagents and adjuvants required for an analysis. Although a test kit consists in most cases of several units, one-piece analysis elements are also available, which must likewise be regarded as testing kits.

As described herein, said kit is suitable for determining the levels of VTN in a biological sample, preferably a urine sample as described herein above, wherein said kit comprises:
a) a reagent for the quantification of VTN;
b) optionally, a reagent for the quantification of a control protein;
c) optionally, further comprising instructions for the use of said reagents in determining the levels of said proteins in a biological sample isolated from a subject.

In preferred embodiments, said kit comprises:
a) an affinity reagent for VTN, preferably for SEQ ID NO: 5;
b) optionally, an affinity reagent for a control protein;
c) optionally, further comprising instructions for the use of said reagents in determining the levels of said proteins in a biological sample isolated from a subject.

Said reagents in points i) to iii) or a) and b), as well as preferred features and embodiments may be as described herein above for the methods of the invention.

The term "an affinity reagent for" as used herein refers to an affinity reagent capable of specifically binding to the recited target protein. The various affinity reagents may be labelled with the same or different tags. Preferably, these will be labelled with different tags for multiplex analysis.

In preferred embodiments, said affinity reagent is a polypeptide, preferably an antibody (e.g. a monoclonal antibody), capable of specifically binding to the recited target proteins.

Numerous methods known to those skilled in the art are available for obtaining antibodies or antigen-binding fragments thereof. Antibodies can be produced using recombinant DNA methods, see, e.g., Current Trends in Monoclonal Antibody Development (Shire et al. 2010). Monoclonal antibodies may also be produced by preparing immortalized cell lines capable of producing antibodies having the desired specificity. Such immortalized cell lines may be produced in a variety of ways.

Conveniently, a small non-human animal, such as a mouse, is hyperimmunized with the desired immunogen. The vertebrate is then sacrificed, usually several days after the final immunization, the spleen cells removed, and the spleen cells immortalized. The most common technique is fusion with a myeloma cell fusion partner, as first described by (Köhler and Milstein 1975)). Other techniques, including EBV transformation, transformation with bare DNA, e.g., oncogenes, retroviruses, etc., or any other method which provides for stable maintenance of the cell line and production of monoclonal antibodies are also well known. Specific techniques for preparing monoclonal antibodies are described in (Harlow and Lane 1988).

Antibodies specifically binding to VTN have been described. Non-limiting examples include the following commercially available antibodies:
- VTN: Cloud Clone corporation: KSB041Hu01 96T

Preferably, said kit comprises a solid support or surface which is coated with an affinity reagent as described herein above (i.e., the primary or capturing affinity reagent). The solid support can include any support known in the art on which said affinity reagent can be immobilized. In some embodiments, said solid supports are microtiter well plates, slides (e.g., glass slides), chips (e.g., protein chips, biosensor chips, such as Biacore chips), microfluidic cartridges, cuvettes, beads (e.g., magnetic beads, xMAP^{®} beads) or resins.

Said reagent for determining the levels of the marker protein as defined herein above may also be a secondary (detection) affinity reagent or antibody. These antibodies can be monoclonal or polyclonal antibodies. Also, these can be derived from any mammalian organism, including mice, rats, hamsters, goats, camels, chicken, rabbit, and others. Typically, the primary antibody and the secondary antibody will be from different animal species. These may also be monoclonal antibodies, with the proviso that, these bind to different epitopes and there is no steric interference. Secondary or detection antibodies can be conjugated to any suitable detection means such as enzymes (e.g., horseradish peroxidase (HRP), alkaline phosphatase (AP), luciferase, and the like) or dyes (e.g., colorimetric dyes, fluorescent dyes, fluorescence resonance energy transfer (FRET)-dyes, time-resolved (TR)-FRET dyes, and the like). Other suitable labels have been described herein above.

Ancillary reagents typically used in an immunoassay, such as a solid support immunoassay, can include, e.g., an immobilization buffer, an immobilization reagent, a dilution buffer, a secondary or detection antibody, a detection reagent, a blocking buffer, a washing buffer, a detection buffer, a stop solution, a system rinse buffer, and a system cleaning solution which are well known by a person skilled in the art.

In some embodiments, said kit for determining the levels of VTN in a biological sample, is a lateral flow immunoassay (LFI) which enables determining when levels of the target protein are above a reference level. The technology as well as typical reagents are as described herein above. To practice the present invention, a large variety of different lateral flow system devices known in the art could be used.

In still a further aspect, the invention relates to the use of a kit as described above in any of the claimed methods, such as a method for the screening, diagnosis or monitoring of kidney fibrosis in a subject; a method for selecting a subject which would likely benefit from the administration of an agent suitable for the preventive and/or therapeutic treatment of fibrosis and a method for determining the prognosis of a subject.

It is contemplated that any features described herein can optionally be combined with any of the embodiments of any of the methods, kits or uses of the invention; and any embodiment discussed in this specification can be implemented with respect to any of these. It will be understood that particular embodiments described herein are shown by way of illustration and not as limitations of the invention.

The use of the word "a" or "an" may mean "one," but it is also consistent with the meaning of "one or more," "at least one," and "one or more than one". The use of the term "another" may also refer to one or more. The use of the term "or" in the claims is used to mean "and/or" unless explicitly indicated to refer to alternatives only or the alternatives are mutually exclusive.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "comprises" also encompasses and expressly discloses the terms "consists of" and "consists essentially of". As used herein, the phrase "consisting essentially of" limits the scope of a claim to the specified materials or steps and those that do not materially affect the basic and novel characteristic(s) of the claimed invention. As used herein, the phrase "consisting of" excludes any element, step, or ingredient not specified in the claim except for, e.g., impurities ordinarily associated with the element or limitation.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

As used herein, words of approximation such as, without limitation, "about", "around", "approximately" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by ±1, 2, 3, 4, 5, 6, 7, 8, 9, or 10%. Accordingly, the term "about" may mean the indicated value ± 5% of its value, preferably the indicated value ± 2% of its value, most preferably the term "about" means exactly the indicated value (± 0%).

The following examples serve to illustrate the present invention and should not be construed as limiting the scope thereof.

### EXAMPLES

The assays disclosed in the Examples below were carried out using the following materials and methodologies.

### Patients and study design

The study workflow is depicted in Figure 6A. Urine samples were collected from kidney transplanted patients from two independent cohorts at the moment of biopsy. For the discovery phase (n=23 patients), clinically indicated biopsies were used (i.e., further to the finding of altered clinical parameters, specifically., serum creatinine and proteinuria). For the validation phase (n=41), patients were recruited from a different hospital and protocol biopsies were used. All procedures performed in studies involving human participants were in accordance with the ethical standards of the Ethical Committee of "Germans Trias i Pujol" Hospital and with the 1964 Helsinki declaration (Lynöe et al. 1991) Informed consent was obtained from all individual participants included in the study.

Both the Discovery and the Validation phases, histopathological diagnosis was used as the criteria to define four groups of patients: normal kidney function (no alteration on clinical parameters), interstitial fibrosis and tubular atrophy (IFTA), acute cellular rejection (ACR) and calcineurin inhibitors toxicity (CNIT). In particular, the following groups of patients were defined:
Renal transplanted patients with Normal Kidney Function (referred as **NKFs**): living or cadaveric donors transplanted patients, with more than 6 months of evolution, Creatinine levels < 1.1 mg/dl, estimated GFR >80 ml/min/1,72 m² and no analytical evidence of proteinuria or haematuria. No biopsies were performed in these patients as there was no clinical indication.
Renal transplanted patients with transplanted kidney dysfunction (referred as "Pathological **patients**"): living or cadaveric donors transplanted patients. All these patients were biopsied by clinical indication (alteration of creatinine levels, mean 2.72 mg/dl at the time of the biopsy). In addition, all patients presented proteinuria.

Kidney biopsies of these patients had specific histological lesions on the pathological examination, which were scored following the Banff criteria (Kim Solez et al. 1993; revisited: Haas et al. 2018). According to the pathologist results, three groups of pathological patients were considered:
1) **IFTA** (Interstitial Fibrosis/Tubular Atrophy): These patients showed different degrees of interstitial fibrosis / tubular atrophy in their biopsies, with scarce or absent local inflammation. Cd4 staining negative.
2) **ACR** (Acute Cellular Rejection): These patients showed different levels of interstitial inflammation and arteritis. Biopsies showed < 10% grade of IFTA lesions or no evidence of fibrotic lesions, <10% glomerular sclerosis, and were C4d and SV40 staining negative.
3) **CNIT** (Calcineurin Inhibitors Toxicity): This group of patients with CNIT presented as main pathological finding a severe hyaline arteriolopathy and/or isometric vacuolization of the tubular epithelium, with scarce or negative inflammation or IFTA areas, and with negative staining for C4d.

**Table 1 (see below) provides the ci ct results of the biopsy according to BANFF score for each patient:**

| **Patient** | **Biopsy diagnosis** | **ci** | **ct** | **Mean** cict |
|---|---|---|---|---|
| **IFTA_08** | IFTA Grade 2 | 2 | 2 | 2 |
| **IFTA_09** | IFTA Grade 1 | 1 | 1 | 1 |
| **IFTA_10** | IFTA Grade 1 | 1 | 1 | 1 |
| **IFTA_11** | IFTA Grade 2 | 2 | 2 | 2 |
| **IFTA_12** | IFTA Grade 2 | 2 | 2 | 2 |
| **ACR_13** | ACR IIA | 2 | 2 | 2 |
| **ACR_14** | ACR | 1 | 1 | 1 |
| **ACR_15** | ACR IIA | 0 | 0 | 0 |
| **ACR_16** | ACR | 0 | 0 | 0 |
| **ACR_17** | ACR IIA | 1 | 1 | 1 |
| **ACR_18** | ACR | 0 | 0 | 0 |
| **CNIT_19** | CNIT | 0 | 0 | 0 |
| **CNIT_20** | CNIT | 1 | 1 | 1 |
| **CNIT_21** | CNIT | 2 | 2 | 2 |
| **CNIT_22** | CNIT | 1 | 1 | 1 |
| **CNIT_23** | CNIT | 0 | 0 | 0 |
| **vNKF_24** | No evidence of rejection | 0 | 1 | 0.5 |
| **vNKF_25** | No evidence of rejection | 1 | 1 | 1 |
| **vNKF_26** | No evidence of rejection | 0 | 0 | 0 |
| **vNKF_27** | No evidence of rejection | 0 | 0 | 0 |
| **vNKF_28** | No evidence of rejection | nd (is) | nd (is) | nd (is) |
| **vNKF_29** | No evidence of rejection | 0 | 0 | 0 |
| **vNKF_30** | No evidence of rejection | 1 | 1 | 1 |
| **vNKF_31** | No evidence of rejection | 0 | 1 | 0.5 |
| **vNKF_32** | No evidence of rejection | 0 | 0 | 0 |
| **vNKF_33** | No evidence of rejection | nd (is) | nd (is) | nd (is) |
| **vIFTA_34** | IFTA Grade 2 | 2 | 2 | 2 |
| **vIFTA_35** | IFTA Grade 2 | 2 | 2 | 2 |
| **vIFTA_36** | IFTA Grade 1 | 1 | 1 | 1 |
| **vIFTA_37** | IFTA Grade 2 | 2 | 2 | 2 |
| **vIFTA_38** | IFTA Grade 2 | 2 | 2 | 2 |
| **vIFTA_39** | IFTA Grade 2 | 2 | 2 | 2 |
| **vIFTA_40** | IFTA Grade 2 | 2 | 2 | 2 |
| **vIFTA_41** | IFTA Grade 3 | 3 | 3 | 3 |
| **vIFTA_42** | IFTA Grade 3 | 3 | 3 | 3 |
| **vIFTA_43** | IFTA Grade 3 | 3 | 3 | 3 |
| **vIFTA_44** | IFTA Grade 3 | 3 | 2 | 2.5 |
| **vACR_45** | ACR IB | nd (is) | nd (is) | nd (is) |
| **vACR_46** | ACR IA | 1 | 1 | 1 |
| **vACR_47** | ACR IB | 1 | 1 | 1 |
| **vACR_48** | ACR IA | 1 | 1 | 1 |
| **vACR_49** | ACR IB, IFTA Grade 2 | 2 | 1 | 1.5 |
| **vACR_50** | ACR | 1 | 1 | 1 |
| vACR_51 | ACR | 0 | 0 | 0 |
| **vACR_52** | ACR IA | nd (is) | nd (is) | nd (is) |
| **vACR_53** | ACR IB | 1 | 1 | 1 |
| **vACR_54** | ACR IA, IFTA Grade 1 | 1 | 1 | 1 |
| **vCNIT_55** | CNIT | 2 | 2 | 2 |
| **vCNIT_56** | CNIT | 2 | 2 | 2 |
| **vCNIT_57** | CNIT | 0 | 1 | 0.5 |
| **vCNIT_58** | CNIT | 1 | 1 | 1 |
| **vCNIT_59** | CNIT | nd (is) | nd (is) | nd (is) |
| **vCNIT_60** | CNIT | 2 | 2 | 2 |
| **vCNIT_61** | CNIT | 1 | 1 | 1 |
| **vCNIT_62** | CNIT | 1 | 0 | 0.5 |
| **vCNIT_63** | CNIT | 2 | 2 | 2 |
| **vCNIT_64** | CNIT | nd (is) | nd (is) | nd (is) |

| | | | | |
|---|---|---|---|---|
| ci, chronic interstitial lesions; ct, chronic tubular lesions; nd (is) not determined (insufficient sample). | | | | |

### Isolation of EV from urine by size-exclusion chromatography

Urinary extracellular vesicles (uEV) were isolated and characterized as described previously by our group using size-exclusion chromatography (SEC) (Lozano-Ramos et al. 2015; Monguió-Tortajada, Morón-Font, et al. 2019). Briefly, 140 ml of each frozen urine sample were thawed overnight at 4°C before being centrifuged at 17,000xg for 10 min. In order to remove large uromodulin protein polymers, the pellet was treated with DTT (200 mg/ml, Sigma-Aldrich) for 10 min at 37°C and it was mixed with the supernatant before centrifuging again at 17,000xg for 10 min (Fernández-Llama et al. 2010) The resulting supernatant was concentrated using a 100 kDa cut-off Centricon filter unit (Millipore, Bedford, MA) until 1 to 2 ml. Then, one ml of the concentrate was loaded onto 12 mL sepharose CL-2B (Sigma-Aldrich, St. Louis, MO, USA) SEC columns and eluted with phosphate-buffered saline (PBS) to collect up to twenty 500 µl fractions.

An optimized protocol for the preparation of concentrated urine before SEC was used for the validation cohort samples, as described in (Puhka et al. 2017). The initial volume of urine was 40 ml, which were centrifuged at 1,800xg for 10 min at 4°C, the pellet was discarded and the supernatant was diluted 1/4 with Tris-EDTA buffer (20 mM, pH=9). After a 90 seconds vortex, the samples were centrifuged at 8,000xg for 15 min at 4°C and the supernatant was concentrated using a 100 kDa cut-off Centricon filter unit. SEC was performed similarly as mentioned previously, but charging a concentrate volume of 150 µl onto 1 ml sepharose columns, from which twenty 100 µl fractions were collected (Fig. 6B).

### uEV-enriched fractions determination

Next, we performed beads-based assay flow cytometry of fractions 7 to 14 to determine the three most EV-enriched fractions. A 10% of SEC fractions volume (50 µl) was incubated with 4 µm aldehyde/sulphate-latex beads (Invitrogen, Carlsbad, CA) for 15 min at room temperature and blocked with BCB buffer (PBS, 0.1 % BSA and 0.01% NaN3, Sigma Aldrich) overnight at room temperature on rotation. After washing with BCB, EV-coated beads were incubated at 4°C for 30 min with antibodies against the tetraspanin markers CD9 and CD63 (clones VJ1/20 and TEA 3/18, respectively), - which are described to be EV markers - using goat-anti-mouse-FITC as secondary antibody (SouthernBiotech, Birmingham, AL) and polyclonal mouse IgG as control isotype (Abcam, Cambrige, UK). In the flow cytometry analysis (FacsVerse, BD Biosciences San Jose, CA), 10,000 beads were acquired and FITC median fluorescence intensity (MFI) of singlet beads were measured for each fraction with FlowJo software (Tree Star, Ashland, OR). For all samples, the three fractions with highest tetraspanins content (uEV-enriched) were pooled and used for further experiments. Protein elution SEC was determined by reading absorbance of 2 µl of each fraction at 280 nm with Nanodrop ND-1000 (Thermo Scientific). In all cases, uEV eluted well before the bulk of soluble proteins (much smaller) started to elute (typically in fractions 6 to 8 or fractions 11 to 19, respectively) (Fig.6B).

### Mass spectrometry analysis and data analysis

### Discovery proteomics

For the discovery phase, 500 µl of uEV-enriched fractions from SEC were analyzed by shot gun proteomics with liquid chromatography followed by mass spectrometry (LC-MS/MS) on an Orbitrap XL. Samples were injected with a 120 min gradient CID method using a 12 cm column after in solution-digestion with LysC and Trypsin (Sigma-Aldrich). As quality controls, BSA solutions were included in the enzymatic digestion and in the LC-MS/MS analysis. Data was analyzed using the Proteome Discoverer software version 2.0 (Thermo Fisher Scientific) and proteins were identified using Mascot (Matrix Science, London UK) against SwissProt human database (UniProt Apr 2015) with a false discovery rate (FDR) of 5%.

Raw data files derived from the MS analysis in the discovery phase were processed using MaxQuant software (Cox and Mann 2008) (version 1.5.3.30) and Uniprot human database (downloaded on December 2015, 70,076 proteins). Maximum FDR was set at 1%, so only those proteins with a q-value lower than 0.01 were kept. Other parameters set in the software include: (i) minimum peptide length of 7; (ii) minimum peptides per protein of 1 and minimum unique peptides per protein of 0; (iii) minimum score for modified peptides of 40; (iv) main search error was set to 4 ppm; (v) cysteine carbamidomethylation was established as a fixed modification and methionine oxidation and acetylation of the N-terminus were established as variable modifications, with a maximum number of modifications per peptide set to 5. Proteins identified as potential contaminants, those only identified by site or identified with a reverse sequence were discarded, as well as proteins with less than 2 unique peptides. All the analyses were thereafter performed with intensity-based absolute quantification (iBAQ) values which were normalized with the EV marker ezrin.

### Targeted proteomics

The selection of proteins for the validation phase was done taking into account statistical differences, the levels of expression of each protein across the samples, the biological significance as well as the feasibility of detection using techniques applicable in the clinics. A known quantity of isotypic heavily labeled standard peptides (ThermoFisher) were spiked into the samples prior to an in-gel trypsin digestion for LC-MS/MS analysis.

For each protein, two unique peptides were selected given that they 1) had a length between 6 and 25 amino acid residues, 2) did not present methionine or tryptophan residues to avoid oxidative modifications and consequent changes in the mass, 3) did not have versions with missed cleavages and 4) did not present two cysteine residues together. At the end of the selection process, 46 unique peptides (for 23 proteins) were searched with targeted mass spectrometry, a technique based on Selected Reaction Monitoring (SRM).

Raw data was processed with the Skyline software (MacLean et al. 2010). Ratios between the unlabeled endogenous peptide and the labeled internal standard were used to calculate endogenous peptide quantity in each sample. Measured values were normalized by the peptide abundance across samples and by endogenous ezrin for each sample.

### Enzyme-linked immunosorbent assay (ELISA)

Up to two milliliters of urine were concentrated to 100 µL using an Amicon Ultra filter unit of 50 kDa cut-off at 2,000x *g* for 20 minutes and analyzed in 96-wells plates with a vitronectin ELISA kit (Cloud-Clone Corporation, USA), following the protocol recommended by the manufacturer. A standard curve provided in the kit was used to calculate vitronectin concentration in samples. Plasma samples at 1:100 dilution from healthy donors were used as positive controls.

### Statistical analysis of proteomics data

Enrichment analyses of gene ontology (GO) - cellular components were performed using the FunRich software (Pathan et al. 2015; 2017). Funrich uses hypergeometric distribution test to check the statistical significance of enriched and depleted terms and corrects for multiple testing with Bonferroni and Benjamini-Hochberg (false discovery rate, FDR) method.

The number of shared proteins was calculated with the online tool InteractiVenn (Heberle et al. 2015).

The Perseus software (Tyanova et al. 2016) (v1.5.6.0 in the discovery phase or v1.6.1.3 in the validation phase) was used to perform a principal component analysis (PCA) and volcano plot.

Protein expression representation and other statistical tests were performed using GraphPad Prism software (v6.0 GraphPad Software, San Diego, CA). After testing for normality, two-sided unpaired t-test (parametric) or Mann-Whitney (non-parametric) were used for the comparison of two groups of samples, and in the case of multiple groups comparison, one-way ANOVA with Holm-Sidak's multiple comparison (parametric), or Kruskall-Wallis with Dunn's multiple comparison test (non-parametric).

Finally, Gene set enrichment analysis software (GSEA v3.0, Broad Institute, Cambridge, MA) (Subramanian et al. 2005) was used to compare enriched gene sets. The gene sets annotated by Geno Ontology (GO) biological process were downloaded from GSEA Molecular Signatures Database (MSigDB v6.2, Broad Institute, Cambridge, MA) (Liberzon et al. 2011). Analyses were performed using the default parameters of GSEA, with 1,000 gene set permutations. False discovery rate (FDR) q-values <0.25 were considered significant as recommended by GSEA software.

### Example 1.- Discovery Phase

### Characterization of uEV-enriched fractions proteome

Urinary-EV from each sample were enriched using SEC. The proteome of uEV was analyzed using mass spectrometry, a total of 1,121 proteins were identified at FDR<1%. After strict filtering of these sequences (as described in the methods section), up to 777 proteins were confidently identified. EV-specific proteins such as Ezrin, CD9 and CD81 tetraspanins and annexin and 14-3-3 families were found in almost all samples. FunRich gene ontology - cellular component enrichment analysis reported that the most enriched was "Exosomes", followed by "Extracellular", "Extracellular region" and "Lysosome", all with a p-value<0.001 (data not shown).

### Number of different proteins in the uEV fraction per patient group

When analyzing the number of different proteins in each group under study, it was found that NKF group presented the lowest number of different proteins in their uEV (396), significantly less than ACR (663) and CNIT (621), as illustrated in Figure 1. Besides, NFK had also the lowest number of proteins common to all individuals classified within this group (27). Of note, there were 15 proteins (1.93%) shared between all samples (Figure 7).

### The transplanted kidney pathology is reflected in uEV proteome

The first aim of the study was to determine if uEV enriched fractions from patients suffering from different kidney pathologies after transplantation have different proteome content. After raw data processing, we performed a principal component analysis (PCA) to visualize samples clustering according to their proteomic profile. As shown in Figure 8, NKF samples were separated from the rest of the samples. IFTA samples were also segregated from ACR and CNIT, which constituted the third cluster. This indicated that changes happening in the kidney when rejection is occurring affect the proteomic content of uEV. In order to find which proteins were dysregulated in kidney graft rejection, we performed a volcano plot (Figure 2). Seven proteins were significantly more expressed in the NKF group, while 48 proteins were significantly more expressed in the pathological group. Six of these proteins presented significantly lower expression in the NKF group, in particular Cathepsin-D (CTSD), Retinol binding protein 4 (RBP4), Cystatin-C (CST3), Vitronectin (VTN), Antithrombin-Ill (SERPINC1) and Angiotensin-converting enzyme 2 (ACE2).

### Example 2.- Validation Phase - Proteins differentiating NKF from pathological kidneys

A second cohort of patients from a different hospital was used to verify those proteins that presented potential as biomarkers of graft rejection and some pathologies. Twenty-three (23) proteins were selected from the discovery phase, and each of them was searched in each sample by using two unique referenced peptides. Since the sensitivity of targeted mass spectrometry is much higher than the shotgun process, we chose a high-quality threshold for evaluation of peptides and those peptides that could be read in at least 90% of the samples with mass spectrometry were used. Nineteen (19) peptides (41.3%) were found in >90% of the samples. The mean value of each pair of peptides was used as the expression level (fmol/sample) for the corresponding protein.

The results of the targeted proteomics analysis revealed that 6 of the proteins selected from the discovery phase (i.e., CTSD, RBP4, CST3, VTN, SERPINC1 and ACE2) were also over-expressed in the pathological groups compared to NKF group (Figure 3).

Receiver operating characteristic (ROC) curves of CTSD, RBP4 and ACE2 showed. AUC values >0.75 with a significant *p*-value what highlights their potential as biomarkers (Figure 9).

The inventors further analyzed the levels of expression of these six proteins in each of the four groups separately and found that there was a statistically significant association of the levels of any of CTSD, RBP4 or SEPINC1 specifically in those kidney transplanted patients presenting acute cellular rejection (Figure 4).

### Example 3.- Vitronectin as biomarker of the degree of kidney fibrosis

When analyzing the levels of expression of these six proteins according to the underlying pathology, the inventors realized that VTN presented a bimodal distribution in the IFTA group (Figure 4, see VTN plot).

In order to decipher the reason, all the clinical parameters and biopsy results were reanalyzed. The unique significant difference between the two subgroups of VTN expression in IFTA patients was the biopsy parameter CICT.

Kidney transplanted patients received their diagnostic from kidney biopsy analyses performed by expert pathologist based on the Banff criteria and according to the hospital protocols. Routinely, the histopathological findings are graded with a clinical score from 0 to 3 following Banff criteria. Two of these criteria refer to the pathological evidence of chronic interstitial lesions and chronic tubular lesions (CICT), both crucial to determine the grade of kidney fibrosis and IFTA (see Table 1 above). When we looked specifically at the CICT score, we found that the levels of VTN enabled to identify three subgroups of patients according to their degree of fibrosis. More specifically, VTN quantification values were discriminating patients with low (<1 cict BANFF) and moderate (1-2 cict BANFF) from severe (>2 cict BANNF) fibrosis as shown in Figure 5.

### Example 4.- Validation of VTN as biomarker of fibrosis with ELISA

The results related to VTN were further assessed by ELISA. With this aim, concentrated urine samples (50 kDa cut-off filter) from a limited number of kidney-transplanted patients with different grades of fibrosis were used in order to optimize the sample processing and ELISA protocol. Samples with CICT score of 0, presented a negligible VTN concentration (Figure 11A). Similar to that observed in targeted proteomics, a ROC curve of the ELISA results showed an AUC of 0.87 (Figure 11B).

### Example 5.- Analysis of VTN in concentrated urine by ELISA in non-kidney-transplanted patients with renal alterations

The ability to use VTN as urine non-invasive biomarker of kidney fibrosis not only in transplanted patients but also in patients suffering from other renal pathologies which may have underlying fibrosis was also explored. With this aim, VTN levels were analyzed by ELISA in concentrated urine samples from patients suffering from diverse kidney alterations (e.g., glomerulosclerosis, amyloidosis, diabetic nephropathy or IgA nephropathy) diagnosed by renal biopsy in native kidneys (not kidney-transplanted) also with different fibrosis grades.

In contrast to kidney-transplanted patients, who were classified following Banff criteria, the level of renal fibrosis of these patients was graded according to the percentage of fibrosis found on the histopathological analysis (by trichrome staining with hematoxylin and eosin, PAS and Silver Methenamine) and following the advice of experienced pathologists, patients were similarly classified into three groups equivalent to Banff CICT <1, 1-2, >2: "mild" (<25% area of fibrosis), "moderate" (25-50% area of fibrosis) and "severe" (>50% area of fibrosis) fibrosis, respectively. The results showed that patients with moderate fibrosis presented significantly higher levels of VTN (p = 0.0056) with respect to patients classified as low (mild) fibrosis (Figure 12A).

Creatine concentration in urine is often used to normalize for protein quantity in urine. In these samples, correction for creatinine concentration further showed a significant difference between mild and severe fibrosis (Figure 12B).

When all data from transplanted and non-transplanted patients was collectively analyzed, the statistical analysis showed that patients with moderate or severe fibrosis presented significantly higher VTN levels than those with mild fibrosis (*p* = 0.0087 and *p* = 0.0200, respectively) (Figure 12C). These values are not normalized by creatinine.

When VTN levels were measured in samples from CKD patients, the results showed that there was a significant difference between mild (<1) and moderate (1-2), and between mild and severe (>2) fibrosis grades, in line with the results in renal transplanted patients shown above. Also, as seen in kidney transplanted patients, high variability was observed in some groups. For instance, some patients showed high levels of VTN in the moderate fibrosis group. This phenomenon may be explained by different reasons. Without willing to be bound by theory, this can be explained by a possible misclassification of the samples due to the intrinsic limitations of the sampling and the evaluation of the renal biopsy - which is estimated to be around 20% (Reeve et al. 2009; Reich et al. 2011; Halloran, Famulski, and Reeve 2016). Indeed, in addition to not being invasive, one of the advantages of the method of the invention with regard to the biopsy is that it may actually more accurately classify patients according to their degree of fibrosis since the analysis is not restricted to a selected area of the kidney but, by determining VTN levels in urine, it provides a global overview on the kidney status.

### References

Al-Awwa, I. A., S. Hariharan, and M. R. First. 1998. 'Importance of Allograft Biopsy in Renal Transplant Recipients: Correlation between Clinical and Histological Diagnosis'. American Journal of Kidney Diseases: The Official Journal of the National Kidney Foundation 31 (6 Suppl 1): S15-18. https://doi.org/10.1053/ajkd.1998.v31.pm9631859.
Alvarez, S., C. Suazo, A. Boltansky, M. Ursu, D. Carvajal, G. Innocenti, A. Vukusich, et al. 2013. 'Urinary Exosomes as a Source of Kidney Dysfunction Biomarker in Renal Transplantation'. Transplantation Proceedings 45 (10): 3719-23. https://doi.org/10.1016/j.transproceed.2013.08.079.
Banta, Scott, Kevin Dooley, and Oren Shur. 2013. 'Replacing Antibodies: Engineering New Binding Proteins'. Annual Review of Biomedical Engineering 15: 93-113. https://doi.org/10.1146/annurev-bioeng-071812-152412.
Chapman, Jeremy R., Philip J. O'Connell, and Brian J. Nankivell. 2005. 'Chronic Renal Allograft Dysfunction'. Journal of the American Society of Nephrology: JASN 16 (10): 3015-26. https://doi.org/10.1681/ASN.2005050463.
Chen, Teresa K., Daphne H. Knicely, and Morgan E. Grams. 2019. 'Chronic Kidney Disease Diagnosis and Management: A Review'. JAMA 322 (13): 1294. https://doi.org/10.1001/jama.2019.14745.
Cosio, F. G., M. El Ters, L. D. Cornell, C. A. Schinstock, and M. D. Stegall. 2016. 'Changing Kidney Allograft Histology Early Posttransplant: Prognostic Implications of 1-Year Protocol Biopsies'. American Journal of Transplantation: Official Journal of the American Society of Transplantation and the American Society of Transplant Surgeons 16 (1): 194-203. https://doi.org/10.1111/ajt.13423.
Cosio, Fernando G., Joseph P. Grande, Timothy S. Larson, James M. Gloor, Jorge A. Velosa, Stephen C. Textor, Matthew D. Griffin, and Mark D. Stegall. 2005. 'Kidney Allograft Fibrosis and Atrophy Early after Living Donor Transplantation'. American Journal of Transplantation: Official Journal of the American Society of Transplantation and the American Society of Transplant Surgeons 5 (5): 1130-36. https://doi.org/10.1111/j.1600-6143.2005.00811.x.
Cosio, Fernando G., Joseph P. Grande, Hani Wadei, Timothy S. Larson, Matthew D. Griffin, and Mark D. Stegall. 2005. 'Predicting Subsequent Decline in Kidney Allograft Function from Early Surveillance Biopsies'. American Journal of Transplantation: Official Journal of the American Society of Transplantation and the American Society of Transplant Surgeons 5 (10): 2464-72. https://doi.org/10.1111/j.1600-6143.2005.01050.x.
Cox, Jürgen, and Matthias Mann. 2008. 'MaxQuant Enables High Peptide Identification Rates, Individualized p.p.b.-Range Mass Accuracies and Proteome-Wide Protein Quantification'. Nature Biotechnology 26 (12): 1367-72. https://doi.org/10.1038/nbt.1511.
Farris AB, Alpers CE. What is the best way to measure renal fibrosis?: A pathologist's perspective. Kidney Int Suppl (2011). 2014 Nov;4(1):9-15. doi: 10.1038/kisup.2014.3. PMID: 26312144; PMCID: PMC4536972.
Fernández-Llama, Patricia, Sookkasem Khositseth, Patricia A. Gonzales, Robert A. Star, Trairak Pisitkun, and Mark A. Knepper. 2010. 'Tamm-Horsfall Protein and Urinary Exosome Isolation'. Kidney International 77 (8): 736-42. https://doi.org/10.1038/ki.2009.550.
Gámez-Valero, Ana, Sara Inés Lozano-Ramos, loana Bancu, Ricardo Lauzurica-Valdemoros, and Francesc E. BorrÃ s. 2015. 'Urinary Extracellular Vesicles as Source of Biomarkers in Kidney Diseases'. Frontiers in Immunology 6 (January). https://doi.org/10.3389/fimmu.2015.00006.
Gheinani, Ali Hashemi, Mike Vögeli, Ulrich Baumgartner, Erik Vassella, Annette Draeger, Fiona C. Burkhard, and Katia Monastyrskaya. 2018. 'Improved Isolation Strategies to Increase the Yield and Purity of Human Urinary Exosomes for Biomarker Discovery'. Scientific Reports 8 (1). https://doi.org/10.1038/s41598-018-22142-x.
Gondos, Adam, Bernd Döhler, Hermann Brenner, and Gerhard Opelz. 2013. 'Kidney Graft Survival in Europe and the United States: Strikingly Different Long-Term Outcomes'. Transplantation Journal 95 (2): 267-74. https://doi.org/10.1097/TP.0b013e3182708ea8.
Greenblatt, David J., Bernard J. Ransil, Jerold S. Harmatz, Thomas W. Smith, David W. Duhme, and Jan Koch-Weser. 1976. 'Variability of 24-Hour Urinary Creatinine Excretion by Normal Subjects'. The Journal of Clinical Pharmacology 16 (7): 321-28. https://doi.org/10.1002/j.1552-4604.1976.tb01527.x*.*
Greiner, M., D. Pfeiffer, and R. D. Smith. 2000. 'Principles and Practical Application of the Receiver-Operating Characteristic Analysis for Diagnostic Tests'. Preventive Veterinary Medicine 45 (1-2): 23-41. https://doi.org/10.1016/s0167-5877(00)00115-x.
Grimm, Paul C., Peter Nickerson, Jim Gough, Rachel McKenna, Elzbieta Stern, John Jeffery, and David N. Rush. 2003. 'Computerized Image Analysis of Sirius Red-Stained Renal Allograft Biopsies as a Surrogate Marker to Predict Long-Term Allograft Function'. Journal of the American Society of Nephrology: JASN 14 (6): 1662-68. https://doi.org/10.1097/01.asn.0000066143.02832.5e.
Haas, M., A. Loupy, C. Lefaucheur, C. Roufosse, D. Glotz, D. Seron, B. J. Nankivell, et al. 2018. 'The Banff 2017 Kidney Meeting Report: Revised Diagnostic Criteria for Chronic Active T Cell-Mediated Rejection, Antibody-Mediated Rejection, and Prospects for Integrative Endpoints for next-Generation Clinical Trials'. American Journal of Transplantation: Official Journal of the American Society of Transplantation and the American Society of Transplant Surgeons 18 (2): 293-307. https://doi.org/10.1111/ajt.14625.
Halloran, Philip F., Konrad S. Famulski, and Jeff Reeve. 2016. 'Molecular Assessment of Disease States in Kidney Transplant Biopsy Samples'. Nature Reviews Nephrology 12 (June): 534.
Harlow, Ed, and David P. Lane. 1988. Antibodies: A Laboratory Manual. Cold Spring Harbor, NY: Cold Spring Harbor Laboratory.
Heberle, Henry, Gabriela Vaz Meirelles, Felipe R da Silva, Guilherme P Telles, and Rosane Minghim. 2015. 'InteractiVenn: A Web-Based Tool for the Analysis of Sets through Venn Diagrams'. BMC Bioinformatics 16 (1). https://doi.org/10.1186/s12859-015-0611-3.
Humphreys, Benjamin D. 2018. 'Mechanisms of Renal Fibrosis'. Annual Review of Physiology 80 (1): 309-26. https://doi.org/10.1146/annurev-physiol-022516-034227.
John, Rohan, Ana Konvalinka, Ana Tobar, Sang J. Kim, Heather N. Reich, and Andrew M. Herzenberg. 2010. 'Determinants of Long-Term Graft Outcome in Transplant Glomerulopathy'. Transplantation 90 (7): 757-64. https://doi.org/10.1097/TP.0b013e3181efcffd.
Keerthikumar, Shivakumar, and Suresh Mathivanan. 2017. 'Proteotypic Peptides and Their Applications'. Methods in Molecular Biology (Clifton, N.J.) 1549: 101-7. https://doi.org/10.1007/978-1-4939-6740-7_8.
Kidney Disease: Improving Global Outcomes (KDIGO) Transplant Work Group. 2009. 'KDIGO Clinical Practice Guideline for the Care of Kidney Transplant Recipients'. American Journal of Transplantation: Official Journal of the American Society of Transplantation and the American Society of Transplant Surgeons 9 Suppl 3 (November): S1-155. https://doi.org/10.1111/j.1600-6143.2009.02834.x.
Köhler, G., and C. Milstein. 1975. 'Continuous Cultures of Fused Cells Secreting Antibody of Predefined Specificity'. Nature 256 (5517): 495-97. https://doi.org/10.1038/256495a0.
Krishnan, Shalini M., Yeong H. Ling, Brooke M. Huuskes, Dorota M. Ferens, Narbada Saini, Christopher T. Chan, Henry Diep, et al. 2019. 'Pharmacological Inhibition of the NLRP3 Inflammasome Reduces Blood Pressure, Renal Damage, and Dysfunction in Salt-Sensitive Hypertension'. Cardiovascular Research 115 (4): 776-87. https://doi.org/10.1093/cvr/cvy252.
Lamb, K. E., S. Lodhi, and H.-U. Meier-Kriesche. 2011. 'Long-Term Renal Allograft Survival in the United States: A Critical Reappraisal'. American Journal of Transplantation: Official Journal of the American Society of Transplantation and the American Society of Transplant Surgeons 11 (3): 450-62. https://doi.org/10.1111/j.1600-6143.2010.03283.x.
Lange, Vinzenz, Paola Picotti, Bruno Domon, and Ruedi Aebersold. 2008. 'Selected Reaction Monitoring for Quantitative Proteomics: A Tutorial'. Molecular Systems Biology 4: 222. https://doi.org/10.1038/msb.2008.61.
Liberzon, A., A. Subramanian, R. Pinchback, H. Thorvaldsdottir, P. Tamayo, and J. P. Mesirov. 2011. 'Molecular Signatures Database (MSigDB) 3.0'. Bioinformatics 27 (12): 1739-40. https://doi.org/10.1093/bioinformatics/btr260.
Lo, Denise J., Bruce Kaplan, and Allan D. Kirk. 2014. 'Biomarkers for Kidney Transplant Rejection'. Nature Reviews Nephrology 10 (4): 215-25. https://doi.org/10.1038/nrneph.2013.281.
Löfblom, John, Fredrik Y. Frejd, and Stefan Ståhl. 2011. 'Non-Immunoglobulin Based Protein Scaffolds'. Current Opinion in Biotechnology 22 (6): 843-48. https://doi.org/10.1016/j.copbio.2011.06.002.
López-Guisa, Jesús M., Allen C. Rassa, Xiaohe Cai, Sarah J. Collins, and Allison A. Eddy. 2011. 'Vitronectin Accumulates in the Interstitium but Minimally Impacts Fibrogenesis in Experimental Chronic Kidney Disease'. American Journal of Physiology-Renal Physiology 300 (5): F1244-54. https://doi.org/10.1152/ajprenal.00701.2010.
Lozano-Ramos, Inés, loana Bancu, Anna Oliveira-Tercero, María Pilar Armengol, Armando Menezes-Neto, Hernando A. Del Portillo, Ricardo Lauzurica-Valdemoros, and Francesc E. Borràs. 2015. 'Size-Exclusion Chromatography-Based Enrichment of Extracellular Vesicles from Urine Samples'. Journal of Extracellular Vesicles 4 (1): 27369. https://doi.org/10.3402/jev.v4.27369.
Ludwig-Portugall, Isis, Eva Bartok, Ermanila Dhana, Beatrix D. G. Evers, Michael J. Primiano, J. Perry Hall, Bernardo S. Franklin, et al. 2016. 'An NLRP3-Specific Inflammasome Inhibitor Attenuates Crystal-Induced Kidney Fibrosis in Mice'. Kidney International 90 (3): 525-39. https://doi.org/10.1016/j.kint.2016.03.035.
Lynöe, N., M. Sandlund, G. Dahlqvist, and L. Jacobsson. 1991. 'Informed Consent: Study of Quality of Information given to Participants in a Clinical Trial'. BMJ (Clinical Research Ed.) 303 (6803): 610-13.
MacLean, Brendan, Daniela M. Tomazela, Nicholas Shulman, Matthew Chambers, Gregory L. Finney, Barbara Frewen, Randall Kern, David L. Tabb, Daniel C. Liebler, and Michael J. MacCoss. 2010. 'Skyline: An Open Source Document Editor for Creating and Analyzing Targeted Proteomics Experiments'. Bioinformatics (Oxford, England) 26 (7): 966-68. https://doi.org/10.1093/bioinformatics/btq054.
Malmström, Lars, Johan Malmström, Nathalie Selevsek, George Rosenberger, and Ruedi Aebersold. 2012. 'Automated Workflow for Large-Scale Selected Reaction Monitoring Experiments'. Journal of Proteome Research 11 (3): 1644-53. https://doi.org/10.1021/pr200844d.
Marcén, Roberto. 2009. 'Immunosuppressive Drugs in Kidney Transplantation: Impact on Patient Survival, and Incidence of Cardiovascular Disease, Malignancy and Infection'. Drugs 69 (16): 2227-43. https://doi.org/10.2165/11319260-000000000-00000.
Meier-Kriesche, Herwig-Ulf, Jesse D. Schold, and Bruce Kaplan. 2004. 'Long-Term Renal Allograft Survival: Have We Made Significant Progress or Is It Time to Rethink Our Analytic and Therapeutic Strategies?' American Journal of Transplantation: Official Journal of the American Society of Transplantation and the American Society of Transplant Surgeons 4 (8): 1289-95. https://doi.org/10.1111/j.1600-6143.2004.00515.x.
Merion, Robert M., Nathan P. Goodrich, Rachel J. Johnson, Stephen P. McDonald, Graeme R. Russ, Brenda W. Gillespie, and David Collett. 2018. 'Kidney Transplant Graft Outcomes in 379 257 Recipients on 3 Continents'. American Journal of Transplantation 18 (8): 1914-23. https://doi.org/10.1111/ajt.14694.
Mohammed Ali, Zahraa, Tomas Tokar, Ihor Batruch, Shelby Reid, Alexandre Tavares-Brum, Paul Yip, Héloïse Cardinal, et al. 2019. 'Urine Angiotensin II Signature Proteins as Markers of Fibrosis in Kidney Transplant Recipients': Transplantation, February, 1. https://doi.org/10.1097/TP.0000000000002676.
Monguió-Tortajada, Marta, Carolina Gálvez-Montón, Antoni Bayes-Genis, Santiago Roura, and Francesc E. Borràs. 2019. 'Extracellular Vesicle Isolation Methods: Rising Impact of Size-Exclusion Chromatography'. Cellular and Molecular Life Sciences 76 (12): 2369-82. https://doi.org/10.1007/s00018-019-03071-y.
Monguió-Tortajada, Marta, Miriam Morón-Font, Ana Gámez-Valero, Laura Carreras-Planella, Francesc E. Borràs, and Marcella Franquesa. 2019. 'Extracellular-Vesicle Isolation from Different Biological Fluids by Size-Exclusion Chromatography'. Current Protocols in Stem Cell Biology, January, e82. https://doi.org/10.1002/cpsc.82.
Naesens, Maarten, Dirk R. J. Kuypers, Katrien De Vusser, Pieter Evenepoel, Kathleen Claes, Bert Bammens, Björn Meijers, et al. 2014. 'The Histology of Kidney Transplant Failure: A Long-Term Follow-up Study'. Transplantation 98 (4): 427-35. https://doi.org/10.1097/TP.0000000000000183.
Nankivell, B. J., C. A. Fenton-Lee, D. R. Kuypers, E. Cheung, R. D. Allen, P. J. O'Connell, and J. R. Chapman. 2001. 'Effect of Histological Damage on Long-Term Kidney Transplant Outcome'. Transplantation 71 (4): 515-23. https://doi.org/10.1097/00007890-200102270-00006.
Pape, Lars, Thomas Henne, Gisela Offner, Juergen Strehlau, Jochen H. H. Ehrich, Michael Mengel, and Paul C. Grimm. 2003. 'Computer-Assisted Quantification of Fibrosis in Chronic Allograft Nephropaty by Picosirius Red-Staining: A New Tool for Predicting Long-Term Graft Function'. Transplantation 76 (6): 955-58. https://doi.org/10.1097/01.TP.0000078899.62040.E5.
Pathan, Mohashin, Shivakumar Keerthikumar, Ching-Seng Ang, Lahiru Gangoda, Camelia Y.J. Quek, Nicholas A. Williamson, Dmitri Mouradov, et al. 2015. 'FunRich: An Open Access Standalone Functional Enrichment and Interaction Network Analysis Tool'. PROTEOMICS 15 (15): 2597-2601. https://doi.org/10.1002/pmic.201400515.
Pathan, Mohashin, Shivakumar Keerthikumar, David Chisanga, Riccardo Alessandro, Ching-Seng Ang, Philip Askenase, Arsen O. Batagov, et al. 2017. 'A Novel Community Driven Software for Functional Enrichment Analysis of Extracellular Vesicles Data'. Journal of Extracellular Vesicles 6 (1): 1321455. https://doi.org/10.1080/20013078.2017.1321455.
Picotti, Paola, Oliver Rinner, Robert Stallmach, Franziska Dautel, Terry Farrah, Bruno Domon, Holger Wenschuh, and Ruedi Aebersold. 2010. 'High-Throughput Generation of Selected Reaction-Monitoring Assays for Proteins and Proteomes'. Nature Methods 7 (1): 43-46. https://doi.org/10.1038/nmeth.1408.
Pocsfalvi, Gabriella, Delfin A. A. Raj, Immacolata Fiume, Annalisa Vilasi, Francesco Trepiccione, and Giovambattista Capasso. 2015. 'Urinary Extracellular Vesicles as Reservoirs of Altered Proteins during the Pathogenesis of Polycystic Kidney Disease'. PROTEOMICS - Clinical Applications 9 (5-6): 552-67. https://doi.org/10.1002/prca.201400199.
Puhka, M., M-E. Nordberg, S. Valkonen, A. Rannikko, O. Kallioniemi, P. Siljander, and T.M. af Hällström. 2017. 'KeepEX, a Simple Dilution Protocol for Improving Extracellular Vesicle Yields from Urine'. European Journal of Pharmaceutical Sciences 98 (February): 30-39. https://doi.org/10.1016/j.ejps.2016.10.021.
Rauh, Manfred. 2012. 'LC-MS/MS for Protein and Peptide Quantification in Clinical Chemistry'. Journal of Chromatography. B, Analytical Technologies in the Biomedical and Life Sciences 883-884 (February): 59-67. https://doi.org/10.1016/j.jchromb.2011.09.030.
Reeve, J., G. Einecke, M. Mengel, B. Sis, N. Kayser, B. Kaplan, and P. F. Halloran. 2009. 'Diagnosing Rejection in Renal Transplants: A Comparison of Molecular- and Histopathology-Based Approaches'. American Journal of Transplantation 9 (8): 1802-10. https://doi.org/10.1111/j.1600-6143.2009.02694.x.
Reich, Heather N., Carol Landolt-Marticorena, Paul C. Boutros, Rohan John, Joan Wither, Paul R. Fortin, Stuart Yang, James W. Scholey, and Andrew M. Herzenberg. 2011. 'Molecular Markers of Injury in Kidney Biopsy Specimens of Patients with Lupus Nephritis'. The Journal of Molecular Diagnostics 13 (2): 143-51. https://doi.org/10.1016/j.jmoldx.2010.10.005.
Reichel, Helmut, Martin Zeier, and Eberhard Ritz. 2004. 'Proteinuria after Renal Transplantation: Pathogenesis and Management'. Nephrology, Dialysis, Transplantation: Official Publication of the European Dialysis and Transplant Association - European Renal Association 19 (2): 301-5. https://doi.org/10.1093/ndt/gfh002.
Reilly, J T et al., "Vitronectin (serum spreading factor): its localisation in normal and fibrotic tissue.", Journal Of Clinical Pathology, vol. 41, no. 12,1 December 1988 (1988-12-01), pages 1269-1272.
Roos-van Groningen, Marian C., Eduard M. Scholten, Patrick M. Lelieveld, Ajda T. Rowshani, Hans J. Baelde, Ingeborg M. Bajema, Sandrine Florquin, et al. 2006. 'Molecular Comparison of Calcineurin Inhibitor-Induced Fibrogenic Responses in Protocol Renal Transplant Biopsies'. Journal of the American Society of Nephrology: JASN 17 (3): 881-88. https://doi.org/10.1681/ASN.2005080891.
Salih, Mahdi, Jeroen A. Demmers, Karel Bezstarosti, Wouter N. Leonhard, Monique Losekoot, Cees van Kooten, Ron T. Gansevoort, Dorien J.M. Peters, Robert Zietse, and Ewout J. Hoorn. 2016. 'Proteomics of Urinary Vesicles Links Plakins and Complement to Polycystic Kidney Disease'. Journal of the American Society of Nephrology 27 (10): 3079-92. https://doi.org/10.1681/ASN.2015090994.
Schwarz, Anke, Michael Mengel, Wilfried Gwinner, Joerg Radermacher, Marcus Hiss, Hans Kreipe, and Hermann Haller. 2005. 'Risk Factors for Chronic Allograft Nephropathy after Renal Transplantation: A Protocol Biopsy Study'. Kidney International 67 (1): 341-48. https://doi. org/10.1111 /j.1523-1755.2005.00087.x.
Sellarés, J., D. G. de Freitas, M. Mengel, J. Reeve, G. Einecke, B. Sis, L. G. Hidalgo, K. Famulski, A. Matas, and P. F. Halloran. 2012. 'Understanding the Causes of Kidney Transplant Failure: The Dominant Role of Antibody-Mediated Rejection and Nonadherence: Attributing Causes of Kidney Transplant Loss'. American Journal of Transplantation 12 (2): 388-99. https://doi.org/10.1111/j.1600-6143.2011.03840.x.
Serón, Daniel, Francesc Moreso, Xavier Fulladosa, Miguel Hueso, Marta Carrera, and Josep M. Grinyó. 2002. 'Reliability of Chronic Allograft Nephropathy Diagnosis in Sequential Protocol Biopsies'. Kidney International 61 (2): 727-33. https://doi.org/10.1046/j.1523-1755.2002.00174.x.
Servais, A., V. Meas-Yedid, L. H. Noël, F. Martinez, C. Panterne, H. Kreis, J. Zuber, et al. 2011. 'Interstitial Fibrosis Evolution on Early Sequential Screening Renal Allograft Biopsies Using Quantitative Image Analysis'. American Journal of Transplantation: Official Journal of the American Society of Transplantation and the American Society of Transplant Surgeons 11 (7): 1456-63. https://doi.org/10.1111/j.1600-6143.2011.03594.x.
Servais, A., V. Meas-Yedid, O. Toupance, Y. Lebranchu, A. Thierry, B. Moulin, I. Etienne, et al. 2009. 'Interstitial Fibrosis Quantification in Renal Transplant Recipients Randomized to Continue Cyclosporine or Convert to Sirolimus'. American Journal of Transplantation: Official Journal of the American Society of Transplantation and the American Society of Transplant Surgeons 9 (11): 2552-60. https://doi.org/10.1111/j.1600-6143.2009.02803.x.
Shamseddin, M. Khaled, and Greg A. Knoll. 2011. 'Posttransplantation Proteinuria: An Approach to Diagnosis and Management'. Clinical Journal of the American Society of Nephrology: CJASN 6 (7): 1786-93. https://doi.org/10.2215/CJN.01310211.
Shao, Bo-Zong, Zhe-Qi Xu, Bin-Ze Han, Ding-Feng Su, and Chong Liu. 2015. 'NLRP3 Inflammasome and Its Inhibitors: A Review'. Frontiers in Pharmacology 6 (November). https://doi.org/10.3389/fphar.2015.00262.
Shire, Steven J., Wayne Gombotz, Karoline Bechtold-Peters, and James Andya, eds. 2010. Current Trends in Monoclonal Antibody Development and Manufacturing. New York, NY: Springer New York. https://doi.org/10.1007/978-0-387-76643-0.
Solez, K., F. Vincenti, and R. S. Filo. 1998. 'Histopathologic Findings from 2-Year Protocol Biopsies from a U.S. Multicenter Kidney Transplant Trial Comparing Tarolimus versus Cyclosporine: A Report of the FK506 Kidney Transplant Study Group'. Transplantation 66 (12): 1736-40. https://doi.org/10.1097/00007890-199812270-00029.
Solez, Kim, Roy A. Axelsen, Hallgrimur Benediktsson, James F. Burdick, Arthur H. Cohen, Robert B. Colvin, Byron P. Croker, Dominique Droz, Michael S. Dunnill, and Philip F. Halloran. 1993. 'International Standardization of Criteria for the Histologic Diagnosis of Renal Allograft Rejection: The Banff Working Classification of Kidney Transplant Pathology'. Kidney International 44 (2): 411-22.
Subramanian, A., P. Tamayo, V. K. Mootha, S. Mukherjee, B. L. Ebert, M. A. Gillette, A. Paulovich, et al. 2005. 'Gene Set Enrichment Analysis: A Knowledge-Based Approach for Interpreting Genome-Wide Expression Profiles'. Proceedings of the National Academy of Sciences 102 (43): 15545-50. https://doi.org/10.1073/pnas.0506580102.
Tyanova, Stefka, Tikira Temu, Pavel Sinitcyn, Arthur Carlson, Marco Y Hein, Tamar Geiger, Matthias Mann, and Jürgen Cox. 2016. 'The Perseus Computational Platform for Comprehensive Analysis of (Prote)Omics Data'. Nature Methods 13 (9): 731-40. https://doi.org/10.1038/nmeth.3901.
Ucar, Ali Riza, Erol Demir, and Mehmet Sukru Sever. 2018. 'Transplant Patients With Failing Renal Allografts'. Experimental and Clinical Transplantation: Official Journal of the Middle East Society for Organ Transplantation 16 Suppl 1 (Suppl 1): 4-8. https://doi.org/10.6002/ect.TOND-TDTD2017.L26.
Uzozie, Anuli Christiana, Nathalie Selevsek, Asa Wahlander, Paolo Nanni, Jonas Grossmann, Achim Weber, Federico Buffoli, and Giancarlo Marra. 2017. 'Targeted Proteomics for Multiplexed Verification of Markers of Colorectal Tumorigenesis'. Molecular & Cellular Proteomics 16 (3): 407-27. https://doi.org/10.1074/mcp.M116.062273.
Vanhove, Thomas, Roel Goldschmeding, and Dirk Kuypers. 2017. 'Kidney Fibrosis: Origins and Interventions'. Transplantation 101 (4): 713-26. https://doi.org/10.1097/TP.0000000000001608.
Weidle, Ulrich H., Georg Tiefenthaler, Elisabeth H. Weiss, Guy Georges, and Ulrich Brinkmann. 2013. 'The Intriguing Options of Multispecific Antibody Formats for Treatment of Cancer'. Cancer Genomics & Proteomics 10 (1): 1-18.
Weaver, Virginia M, Dennis J Kotchmar, Jeffrey J Fadrowski, and Ellen K Silbergeld. 2016. 'Challenges for Environmental Epidemiology Research: Are Biomarker Concentrations Altered by Kidney Function or Urine Concentration Adjustment?' Journal of Exposure Science & Environmental Epidemiology 26 (1): 1-8. https://doi.org/10.1038/jes.2015.8.
Williams, Winfred W., Diana Taheri, Nina Tolkoff-Rubin, and Robert B. Colvin. 2012. 'Clinical Role of the Renal Transplant Biopsy'. Nature Reviews Nephrology 8 (2): 110-21. https://doi.org/10.1038/nrneph.2011.213.
Xu, Shengchao, Xizhe Li, Yuanqi Liu, Yu Xia, Ruimin Chang, and Chunfang Zhang. 2019. 'Inflammasome Inhibitors: Promising Therapeutic Approaches against Cancer'. Journal of Hematology & Oncology 12 (1). https://doi.org/10.1186/s13045-019-0755-0.
Yáñez-Mó, Maria, Pia R.-M. Siljander, Zoraida Andreu, Apolonija Bedina Zavec, Francesc E. Borràs, Edit I. Buzas, Krisztina Buzas, et al. 2015. 'Biological Properties of Extracellular Vesicles and Their Physiological Functions'. Journal of Extracellular Vesicles 4 (1): 27066. https://doi.org/10.3402/jev.v4.27066.
Zahid, Ayesha, Bofeng Li, Arnaud John Kombe Kombe, Tengchuan Jin, and Jinhui Tao. 2019. 'Pharmacological Inhibitors of the NLRP3 Inflammasome'. Frontiers in Immunology 10 (October). https://doi.org/10.3389/fimmu.2019.02538.
Zhang, Wei, Xiangjun Zhou, Qisheng Yao, Yutao Liu, Hao Zhang, and Zheng Dong. 2017. 'HIF-1-Mediated Production of Exosomes during Hypoxia Is Protective in Renal Tubular Cells'. American Journal of Physiology. Renal Physiology 313 (4): F906-13. https://doi.org/10.1152/ajprenal.00178.2017.
Zweig, M. H., and G. Campbell. 1993. 'Receiver-Operating Characteristic (ROC) Plots: A Fundamental Evaluation Tool in Clinical Medicine'. Clinical Chemistry 39 (4): 561-77.

## Claims

1. A method for the screening, diagnosis or monitoring of kidney fibrosis in a subject, wherein said method comprises or consists of:
a. determining vitronectin (VTN) protein levels in a urine sample from said subject; and
b. determining whether the VTN protein levels in the sample are increased with respect to a reference value;
wherein increased VTN protein levels in a) with regard to the reference value is indicative of severe or moderate-severe kidney fibrosis.

2. A method for determining the degree of kidney fibrosis in a subject, wherein said method comprises or consist of:
a. determining vitronectin (VTN) protein levels in a urine sample from said subject; and
b. determining whether the VTN protein levels in the sample are increased with respect to a reference value;
wherein increased VTN protein levels in a) with regard to the reference value is indicative of severe or moderate-severe kidney fibrosis.

3. *In vitro* method for classifying a subject according to its degree of kidney fibrosis, said method comprising steps a) and b) of claim 1,
wherein increased VTN protein levels in a urine sample from said subject with respect to a reference value is indicative of severe or moderate-severe kidney fibrosis.

4. The method according to any of claims 1 to 3, wherein said subject has chronic kidney disease.

5. The method according to any of claims 1 to 4, wherein said subject is a kidney transplanted patient.

6. A method for determining the prognosis of a subject on the basis of its degree of kidney fibrosis, wherein said method comprises steps a) and b) of claim 1,
wherein increased VTN protein levels in a) with regard to a reference value is indicative of severe or moderate-severe kidney fibrosis and poor prognosis.

7. A method for selecting a subject who would likely benefit from the administration of an antifibrotic agent, wherein said subject is classified as presenting low or moderate fibrosis according to a method which comprises:
a. determining vitronectin (VTN) protein levels in a urine sample from said subject; and
b. determining whether the VTN protein levels in the sample are decreased with respect to a reference value;
wherein decreased VTN protein levels in a) with regard to the reference value is indicative of low or moderate kidney fibrosis.

8. The method according to any one of claims 1 to 7, wherein said sample is a urine extracellular vesicles (EVs)-enriched sample.

9. The method according to claim 8, wherein said urine extracellular vesicles (EVs)-enriched sample is obtained by concentration of the urine sample.

10. The method according to any one of claims 1 to 9, wherein VTN protein levels are determined by an immunoassay, preferably said immunoassay is selected from the group consisting of an enzyme-linked immunosorbent assay (ELISA), a fluorescent immunosorbent assay (FIA), a chemiluminescence immunoassay (CIA), a radioimmunoassay (RIA), an immunoradiometric assay (IRMA), an enzyme multiplied immunoassay, a solid phase radioimmunoassay (SPROA), a fluorescence polarization (FP) assay, a fluorescence resonance energy transfer (FRET) assay, a time-resolved fluorescence resonance energy transfer (TR-FRET) assay, a surface plasmon resonance (SPR) assay, a bead-based flow-cytometry immunoassay, an electro-chemiluminescent immunoassay (ECL) or a lateral flow immunoassay (LFI).

11. The method according to any one of claims 1 to 9, wherein VTN protein levels are determined by a mass spectrometry-based method, preferably said mass spectrometry-based method is selected from the group consisting of liquid chromatography coupled to mass spectrometry (LC / MS) and tandem LC-MS / MS.

12. The method according to any one of claims 1 to 11, wherein said subject is a human subject.

13. The method according to any one of claims 1 to 12, wherein said method is a computer-implemented method wherein the comparison between the VTN protein levels in the subject sample and the reference value is conducted using a computer.

14. *In vitro* use of VTN protein levels as biomarker of the degree of kidney fibrosis.

15. Use of a kit for determining the levels of VTN protein in a method according to any of claims 1 to 13, wherein said kit comprises:
- a reagent for determining VTN protein levels; and
- optionally, a reagent for determining the levels of a normalizing protein, and
optionally, further comprising instructions for the use of said reagents in determining said proteins expression levels in a urine sample isolated from a subject.

## Patentansprüche

1. Verfahren für das Screening, die Diagnose oder die Überwachung von Nierenfibrose in einem Individuum, wobei das genannte Verfahren Folgendes umfasst oder daraus besteht:
a. das Bestimmen der Proteinspiegel von Vitronektin (VTN) in einer Urinprobe aus dem genannten Individuum; und
b. das Bestimmen, ob die Proteinspiegel von VTN in der Probe in Bezug auf einen Referenzwert erhöht sind;
wobei erhöhte Proteinspiegel von VTN in a) in Bezug auf den Referenzwert auf schwere oder mittelschwere Nierenfibrose schließen lassen.

2. Verfahren für die Bestimmung des Grades von Nierenfibrose in einem Individuum, wobei das genannte Verfahren Folgendes umfasst oder daraus besteht:
a. das Bestimmen der Proteinspiegel von Vitronektin (VTN) in einer Urinprobe aus dem genannten Individuum; und
b. das Bestimmen, ob die Proteinspiegel von VTN in der Probe in Bezug auf einen Referenzwert erhöht sind;
wobei erhöhte Proteinspiegel von VTN in a) in Bezug auf den Referenzwert auf schwere oder mittelschwere Nierenfibrose schließen lassen.

3. In-vitro-Verfahren für das Klassifizieren eines Individuums gemäß dessen Grad von Nierenfibrose, wobei das genannte Verfahren Schritt a) und b) von Anspruch 1 umfasst,
wobei erhöhte Proteinspiegel von VTN in einer Urinprobe aus dem genannten Individuum in Bezug auf einen Referenzwert auf schwere oder mittelschwere Nierenfibrose schließen lassen.

4. Verfahren nach einem der Ansprüche 1 bis 3, wobei das genannte Individuum eine chronische Nierenerkrankung aufweist.

5. Verfahren nach einem der Ansprüche 1 bis 4, wobei das genannte Individuum ein nierentransplantierter Patient ist.

6. Verfahren für die Bestimmung der Prognose eines Individuums basierend auf dessen Grad von Nierenfibrose, wobei das genannte Verfahren Schritt a) und b) von Anspruch 1 umfasst,
wobei erhöhte Proteinspiegel von VTN in a) in Bezug auf einen Referenzwert auf schwere oder mittelschwere Nierenfibrose und eine schlechte Prognose schließen lassen.

7. Verfahren für das Auswählen eines Individuums, welches wahrscheinlich von der Verabreichung eines antifibrotischen Mittels profitieren würde, wobei der genannte Patient als milde oder mittlere Fibrose aufweisend gemäß einem Verfahren klassifiziert wird, welches Folgendes umfasst:
a. das Bestimmen der Proteinspiegel von Vitronektin (VTN) in einer Urinprobe aus dem genannten Individuum; und
b. das Bestimmen, ob die Proteinspiegel von VTN in der Probe in Bezug auf einen Referenzwert verringert sind;
wobei verringerte Proteinspiegel von VTN in a) in Bezug auf den Referenzwert auf milde oder mittlere Nierenfibrose schließen lassen.

8. Verfahren nach einem der Ansprüche 1 bis 7, wobei die genannte Probe eine mit extrazellulären Vesikeln (EV) angereichte Urinprobe ist.

9. Verfahren nach Anspruch 8, wobei die genannte mit extrazellulären Vesikeln (EV) angereichte Urinprobe mittels des Konzentrierens der Urinprobe erhalten wird.

10. Verfahren nach einem der Ansprüche 1 bis 9, wobei die Proteinspiegel von VTN mittels eines Immuntests bestimmt werden, wobei der genannte Immuntest vorzugsweise aus der Gruppe bestehend aus einem enzymgekoppelten Immunadsorptionstest (ELISA), einem Fluoreszenz-Immunadsorptionstest (FIA), einem Chemolumineszenz-Immuntest (CIA), einem Radioimmuntest (RIA), einem immunradiometrischen Test (IRMA), einem enzymverstärktes Immuntest, einem Festphasenradioimmuntest (SPROA), einem Fluoreszenzpolarisations (FP)-Test, einem Fluoreszenz-Resonanz-Energie-Transfer (FRET)-Test, einem zeitaufgelösten Fluoreszenz-Resonanz-Energie-Transfer (TR-FRET)-Test, einem Oberflächen-Plasmonen-Resonanz (SPR)-Test, einem perlenbasierten Durchflusszytometrie-Immuntest, einem Elektrochemolumineszenz (ECL)-Immuntest oder einem Seitenstrom-Immuntest (LFI) ausgewählt wird.

11. Verfahren nach einem der Ansprüche 1 bis 9, wobei die Proteinspiegel von VTN mittels eines massenspektrometriebasierten Verfahrens bestimmt werden, wobei vorzugsweise das genannte massenspektrometriebasierte Verfahren aus der Gruppe bestehend aus Flüssigkeitschromatographie gekoppelt mit Massenspektrometrie (LC/MS) und Tandem-LC-MS/MS ausgewählt wird.

12. Verfahren nach einem der Ansprüche 1 bis 11, wobei das genannte Individuum ein menschliches Individuum ist.

13. Verfahren nach einem der Ansprüche 1 bis 12, wobei das genannte Verfahren ein computerimplementiertes Verfahren ist, in welchem der Vergleich zwischen den Proteinspiegeln von VTN in der Probe des Individuums und dem Referenzwert unter Verwendung eines Computers durchgeführt wird.

14. In-vitro-Verwendung von den Proteinspiegeln von VTN als Biomarker des Grades von Nierenfibrose.

15. Verwendung eines Kits für die Bestimmung der Spiegel des VTN-Proteins in einem Verfahren nach einem der Ansprüche 1 bis 13, wobei das genannte Kit Folgendes umfasst:
- ein Reagenz für die Bestimmung der Proteinspiegel von VTN; und
- wahlweise, ein Reagenz für die Bestimmung der Spiegel eines normalisierenden Proteins, und
wahlweise, zusätzlich umfassend Anweisungen für die Verwendung der genannten Reagenzien bei der Bestimmung der Expressionsniveaus der genannten Proteinen in einer Urinprobe isoliert aus einem Individuum.

## Revendications

1. Procédé pour le dépistage, le diagnostic ou la surveillance de fibrose rénale chez un sujet, dans lequel ledit procédé comprend ou est constitué par :
a. la détermination des niveaux de protéine de vitronectine (VTN) dans un échantillon d'urine dudit sujet ; et
b. la détermination de si les niveaux de protéine de VTN dans l'échantillon sont augmentés par rapport à une valeur de référence ;
dans lequel des niveaux de protéine de VTN augmentés en a) par rapport à la valeur de référence sont indicatifs de fibrose rénale sévère ou modérée-sévère.

2. Procédé pour la détermination du degré de fibrose rénale chez un sujet, dans lequel ledit procédé comprend ou est constitué par :
a. la détermination des niveaux de protéine de vitronectine (VTN) dans un échantillon d'urine dudit sujet ; et
b. la détermination de si les niveaux de protéine de VTN dans l'échantillon sont augmentés par rapport à une valeur de référence ;
dans lequel des niveaux de protéine de VTN augmentés en a) par rapport à la valeur de référence sont indicatifs de fibrose rénale sévère ou modérée-sévère.

3. Procédé *in vitro* pour la classification d'un sujet selon son degré de fibrose rénale, ledit procédé comprenant les étapes a) et b) selon la revendication 1,
dans lequel des niveaux de protéine de VTN augmentés dans un échantillon d'urine dudit sujet par rapport à une valeur de référence sont indicatifs de fibrose rénale sévère ou modérée-sévère.

4. Procédé selon l'une quelconque des revendications 1 à 3, dans lequel ledit sujet souffre de maladie rénale chronique.

5. Procédé selon l'une quelconque des revendications 1 à 4, dans lequel ledit sujet est un patient de transplantation de rein.

6. Procédé pour la détermination du pronostic d'un sujet sur la base de son degré de fibrose rénale, dans lequel ledit procédé comprend les étapes a) et b) selon la revendication 1,
dans lequel des niveaux de protéine de VTN augmentés en a) par rapport à une valeur de référence sont indicatifs de fibrose rénale sévère ou modérée-sévère et de mauvais pronostic.

7. Procédé pour sélectionner un sujet qui est susceptible de bénéficier de l'administration d'un agent antifibrotique, dans lequel ledit sujet est classé comme souffrant fibrose faible ou modérée selon un procédé qui comprend :
a. la détermination des niveaux de protéine de vitronectine (VTN) dans un échantillon d'urine dudit sujet ; et
b. la détermination de si les niveaux de protéine de VTN dans l'échantillon sont réduits par rapport à une valeur de référence ;
dans lequel des niveaux de protéine de VTN réduits en a) par rapport à la valeur de référence sont indicatifs de fibrose rénale faible ou modérée.

8. Procédé selon l'une quelconque des revendications 1 à 7, dans lequel ledit échantillon est un échantillon enrichi en vésicules extracellulaires (EV) d'urine.

9. Procédé selon la revendication 8, dans lequel ledit échantillon enrichi en vésicules extracellulaires (EV) d'urine est obtenu par concentration de l'échantillon d'urine.

10. Procédé selon l'une quelconque des revendications 1 à 9, dans lequel les niveaux de protéine de VTN sont déterminés par le biais d'un immunoessai, de préférence ledit immunoessai est choisi parmi le groupe constitué par un essai d'immunoabsortion lié aux enzymes (ELISA), un essai d'immunoabsortion fluorescent (FIA), un immunoessai de chimioluminescence (CIA), un radioimmunoessai (RIA), un essai immunoradiométrique (IRMA), un immunoessai multiplié par enzymes, un radioimmunoessai en phase solide (SPROA), un essai de polarisation de fluorescence (FP), un essai de transfert d'énergie par résonance en fluorescence (FRET), un essai de transfert d'énergie par résonance en fluorescence de résolution dans le temps (TR-FRET), un essai de résonance de plasmon de surface (SPR), un immunoessai de cytométrie en flux à base de perles, un immunoessai électro-quimioluminescent (ECL) ou un immunoessai à flux latéral (LFI).

11. Procédé selon l'une quelconque des revendications 1 à 9, dans lequel les niveaux de protéine de VTN sont déterminés par le biais d'un procédé à base de spectrométrie de masse, de préférence ledit procédé à base de spectrométrie de masse est choisi parmi le groupe constitué par la chromatographie de liquides couplée à spectrométrie de masse (LC/MS) et LC-MS/MS en tandem.

12. Procédé selon l'une quelconque des revendications 1 à 11, dans lequel ledit sujet est un sujet humain.

13. Procédé selon l'une quelconque des revendications 1 à 12, dans lequel ledit procédé est un procédé mis en œuvre par ordinateur dans lequel la comparaison entre les niveaux de protéine de VTN dans l'échantillon du sujet et la valeur de référence est effectuée en utilisant un ordinateur.

14. Utilisation *in vitro* de niveaux de protéine de VTN comme biomarqueur du degré de fibrose rénale.

15. Utilisation d'un kit pour la détermination des niveaux de protéine de VTN dans un procédé selon l'une quelconque des revendications 1 à 13, dans lequel ledit kit comprend :
- un réactif pour déterminer les niveaux de protéine de VTN ; et
- optionnellement, un réactif pour déterminer les niveaux d'une protéine de normalisation, et
optionnellement, comprenant en outre des instructions pour l'utilisation desdits réactifs dans la détermination desdites niveaux d'expression de protéines dans un échantillon d'urine isolé à partir d'un sujet.
